(19) Europäisches Patentamt
European Patent Office
Office européen des brevets

(11) EP 1 258 474 A2

(12) EUROPEAN PATENT APPLICATION

(43) Date of publication:
**20.11.2002 Bulletin 2002/47**

(51) Int Cl.[7]: **C07C 235/40**, A61K 31/16, A61P 15/10

(21) Application number: **02253114.9**

(22) Date of filing: **02.05.2002**

(84) Designated Contracting States:
**AT BE CH CY DE DK ES FI FR GB GR IE IT LI LU MC NL PT SE TR**
Designated Extension States:
**AL LT LV MK RO SI**

(30) Priority: **14.05.2001 GB 0111709**

(71) Applicants:
- **Pfizer Limited**
  **Sandwich, Kent CT13 9NJ (GB)**
  Designated Contracting States:
  **GB**
- **PFIZER INC.**
  **New York, N.Y. 10017 (US)**
  Designated Contracting States:
  **BE CH DE DK ES FI FR GR IE IT LI LU MC NL PT SE AT CY**

(72) Inventors:
- **Cook, Andrew Simon**
  **Sandwich, Kent CT13 9NJ (GB)**
- **Stobie, Alan**
  **Sandwich, Kent CT13 9NJ (GB)**

(74) Representative: **Rutt, Jason Edward et al**
**Pfizer Limited**
**European Patents Department**
**Ramsgate Road**
**Sandwich, Kent CT13 9NJ (GB)**

# (54) Alkylamide compounds

(57) The invention provides compounds of formula (I) wherein $R^1$ is optionally substituted $C_{1-6}$alkyl, optionally substituted carbocyclyl, optionally substituted heterocyclyl, $C_{1-6}$alkoxy, $-NR^2R^3$ or $-NR^4SO_2R^5$; n is 2 to 6; X is oxygen, sulfur or $-CH_2-$; Y is $C_{1-6}$alkyl which may be branched- or straight-chain, and may be independently substituted by one or more halo, $C_{1-4}$alkoxy, hydroxy or $C_{3-7}$cycloalkyl; wherein the linkage $-(CH_2)_n-$ and the linkage when X is $-CH_2-$, may be substituted by $C_{1-4}$alkyl, $C_{1-4}$haloalkyl, hydroxy, $C_{1-4}$alkoxy, $C_{1-4}$haloalkoxy, hydroxy$C_{1-4}$alkyl, $C_{1-4}$alkoxy$C_{1-4}$alkyl, $C_{1-4}$haloalkoxy$C_{1-4}$alkyl, $C_{3-7}$cycloalkyl or $C_{3-7}$cycloalkyl$C_{1-4}$alkyl. The compounds of the invention are useful in the treatment of *inter alia* sexual dysfunction, particularly female sexual dysfunction.

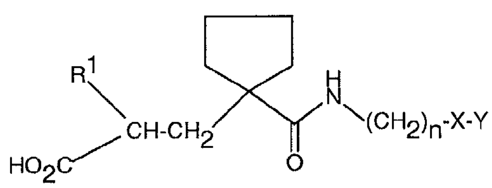

(I)



Printed by Jouve, 75001 PARIS (FR)

## Description

This invention relates to inhibitors of neutral endopeptidase enzyme (NEP), uses thereof, processes for the preparation thereof, intermediates used in the preparation thereof and compositions containing said inhibitors. These inhibitors have utility in a variety of therapeutic including male and female sexual dysfunction, particularly female sexual dysfunction (FSD) especially wherein the FSD is female sexual arousal disorder (FSAD).
NEP inhibitors are disclosed in WO 91/07386 and WO 91/10644.
According to a first aspect, the invention provides a compound of formula (I), pharmaceutically acceptable salts, solvates or prodrugs thereof;

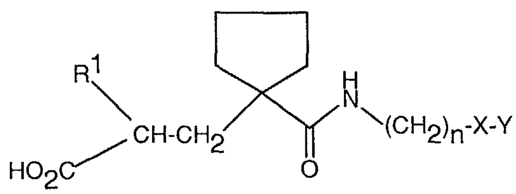

(I)

wherein

$R^1$ is $C_{1-6}$alkyl which may be substituted by one or more substituents, which may be the same or different, selected from the list: halo, hydroxy, $C_{1-6}$alkoxy, $C_{1-6}$hydroxyalkoxy, $C_{1-6}$alkoxy$C_{1-6}$alkoxy, carbocyclyl (preferably $C_{3-7}$cycloalkyl, $C_{3-7}$cycloalkenyl or phenyl), carbocyclyloxy (preferably phenoxy), $C_{1-4}$alkoxycarbocyclyloxy (preferably $C_{1-4}$alkoxyphenoxy), heterocyclyl, heterocyclyloxy, $-NR^2R^3$, $-NR^4COR^5$, $-NR^4SO_2R^5$, $-CONR^2R^3$, $-S(O)_pR^6$, $-COR^7$ and $-CO_2(C_{1-4}$alkyl); or $R^1$ is carbocyclyl (preferably $C_{3-7}$cycloalkyl or phenyl) or heterocyclyl, each of which may be substituted by one or more substituents from said list, which substituents may be the same or different, which list further includes $C_{1-6}$alkyl; or $R^1$ is $C_{1-6}$alkoxy, $-NR^2 R^3$ or $-NR^4SO_2R^5$;
wherein

- $R^2$ and $R^3$ , which may be the same or different, carbocyclyl (preferably $C_{3-7}$cycloalkyl or phenyl) or heterocyclyl (each of which may be substituted by $C_{1-4}$alkyl, hydroxy or $C_{1-4}$alkoxy), or are hydrogen or $C_{1-4}$alkyl; or $R^2$ and $R^3$ together with the nitrogen to which they are attached form a pyrrolidinyl, piperidino, morpholino, piperazinyl or *N*-($C_{1-4}$ alkyl)piperazinyl group;
- $R^4$ is H or $C_{1-4}$alkyl;
- $R^5$ is $C_{1-4}$alkyl, $CF_3$, aryl, ($C_{1-4}$ alkyl)aryl, ($C_{1-4}$alkoxy)aryl, heterocyclyl, $C_{1-4}$alkoxy or $-NR^2R^3$;
- $R^6$ is $C_{1-4}$alkyl, aryl, heterocyclyl or $NR^2R^3$; and
- $R^7$ is $C_{1-4}$alkyl, $C_{3-7}$cycloalkyl, aryl or heterocyclyl;

p is 0, 1, 2 or 3;
n is 2 to 6;
X is oxygen, sulfur or $-CH_2-$;
Y is $C_{1-6}$alkyl which may be branched- or straight-chain, and may be independently substituted by one or more halo, $C_{1-4}$alkoxy, hydroxy or $C_{3-7}$cycloalkyl;
wherein the linkage $-(CH_2)_n-$ and the linkage when X is $-CH_2-$, may be substituted by $C_{1-4}$alkyl, $C_{1-4}$haloalkyl, hydroxy, $C_{1-4}$alkoxy, $C_{1-4}$haloalkoxy, hydroxy$C_{1-4}$alkyl, $C_{1-4}$alkoxy$C_{1-4}$alkyl, $C_{1-4}$haloalkoxy$C_{1-4}$alkyl, $C_{3-7}$cycloalkyl or $C_{3-7}$cycloalkyl$C_{1-4}$alkyl.

Preferably $R^1$ is $C_{1-6}$alkyl, $C_{1-6}$alkoxy, $C_{1-6}$alkoxy($C_{1-3}$)alkyl, $C_{1-6}$alkoxy$C_{1-6}$alkoxy$C_{1-3}$alkyl or $C_{1-6}$alkyl substituted with phenyl, optionally substituted by one or more alkyl, alkoxy, alkylthio, halogen, or phenyl (which phenyl may be independently substituted by one or more alkyl, alkoxy, alkylthio or halogen).
More preferably $R^1$ is $C_{1-6}$alkyl, $C_{1-6}$alkoxy, $C_{1-6}$alkoxy($C_{1-3}$)alkyl (preferably methoxyethyl) or $C_{1-6}$alkoxy$C_{1-6}$alkoxy$C_{1-3}$alkyl (preferably methoxyethoxymethyl).
More preferably still $R^1$ is $C_{1-4}$alkyl (preferably propyl) or $C_{1-6}$alkoxy($C_{1-3}$)alkyl (preferably methoxyalkyl, more preferably methoxyethyl).

Preferred compounds are of formula Ia

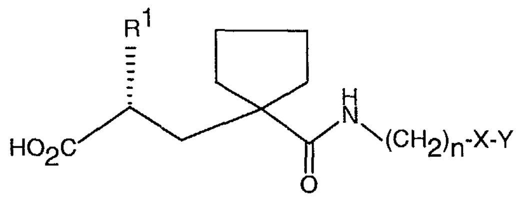

(Ia)

Prefererably X is O or $-CH_2$

Preferably Y is $C_{1-6}$ alkyl, straight chain or branched

Particularly preferred compounds of the invention are:

2-{[1-({[2-butoxy-1-(hydroxymethyl)ethyl]amino}carbonyl)cyclopentyl]methyl}-4-methoxybutanoic acid (Example 2);
4-methoxy-2-({1-[(octylamino)carbonyl]cyclopentyl}methyl)butanoic acid (Example 4);
2-({1-[(heptylamino)carbonyl]cyclopentyl}methyl)-4-methoxybutanoic acid (Example 3);
2-[(-{[(3-butoxypropyl)amino]carbonyl}cyclopentyl)methyl]-4-methoxybutanoic acid (Example 5); and
2-{[1-({[1-hydroxymethyl)heptyl]amino}carbonyl)cyclopentyl]methyl}-4-methoxybutanoic acid (Example 7).

Unless otherwise indicated, any alkyl group may be straight or branched and is of 1 to 6 carbon atoms, preferably 1 to 4 and particularly 1 to 3 carbon atoms.

Unless otherwise indicated, any carbocyclyl group contains 3 to 8 ring-atoms, and may be saturated, unsaturated or aromatic. Preferred saturated carbocyclyl groups are cyclopropyl, cyclopentyl or cyclohexyl. Preferred unsaturated carbocyclyl groups contain up to 3 double bonds. A preferred aromatic carbocyclyl group is phenyl. The term carbocylic should be similarly construed. In addition, the term carbocyclyl includes any fused combination of carbocyclyl groups, for example naphthyl, phenanthryl, indanyl and indenyl.

Unless otherwise indicated, any heterocyclyl group contains 5 to 7 ring-atoms up to 4 of which may be heteroatoms such as nitrogen, oxygen and sulfur, and may be saturated, unsaturated or aromatic. Examples of heterocyclyl groups are furyl, thienyl, pyrrolyl, pyrrolinyl, pyrrolidinyl, imidazolyl, dioxolanyl, oxazolyl, thiazolyl, imidazolyl, imidazolinyl, imidazolidinyl, pyrazolyl, pyrazolinyl, pyrazolidinyl, isoxazolyl, isothiazolyl, oxadiazolyl, triazolyl, thiadiazolyl, pyranyl, pyridyl, piperidinyl, dioxanyl, morpholino, dithianyl, thiomorpholino, pyridazinyl, pyrimidinyl, pyrazinyl, piperazinyl, sulfolanyl, tetrazolyl, triazinyl, azepinyl, oxazepinyl, thiazepinyl, diazepinyl and thiazolinyl. In addition, the term heterocyclyl includes fused heterocyclyl groups, for example benzimidazolyl, benzoxazolyl, imidazopyridinyl, benzoxazinyl, benzothiazinyl, oxazolopyridinyl, benzofuranyl, quinolinyl, quinazolinyl, quinoxalinyl, dihydroquinazolinyl, benzothiazolyl, phthalimido, benzofuranyl, benzodiazepinyl, indolyl and isoindolyl. The term heterocyclic should be similarly construed.

Halo means fluoro, chloro, bromo or iodo.

For the avoidance of doubt, unless otherwise indicated, the term substituted means substituted by one or more defined groups. In the case where groups may be selected from a number of alternative groups, the selected groups may be the same or different.

For the avoidance of doubt, the term independently means that where more than one substituent is selected from a number of possible substituents, those substituents may be the same or different.

The pharmaceutically or veterinarily acceptable salts of the compounds of formula I which contain a basic centre are, for example, non-toxic acid addition salts formed with inorganic acids such as hydrochloric, hydrobromic, hydroiodic, sulfuric and phosphoric acid, with carboxylic acids or with organo-sulfonic acids. Examples include the HCl, HBr, HI, sulfate or bisulfate, nitrate, phosphate or hydrogen phosphate, acetate, benzoate, succinate, saccharate, fumarate, maleate, lactate, citrate, tartrate, gluconate, camsylate, methanesulfonate, ethanesulfonate, benzenesulfonate, p-toluenesulfonate and pamoate salts. Compounds of the invention can also provide pharmaceutically or veterinarily acceptable metal salts, in particular non-toxic alkali and alkaline earth metal salts, with bases. Examples include the sodium, potassium, aluminium, calcium, magnesium, zinc, diolamine, olamine, ethylenediamine, tromethamine, chloine, megulamine and diethanolamine salts. For reviews on suitable pharmaceutical salts see Berge *et al*,

J. Pharm, Sci., 66, 1-19, 1977; P L Gould, International Journal of Pharmaceutics, 33 (1986), 201-217; and Bighley *et al*, Encyclopedia of Pharmaceutical Technology, Marcel Dekker Inc, New York 1996, Volume 13, page 453-497.

The pharmaceutically acceptable solvates of the compounds of the invention include the hydrates thereof.

Hereinafter, compounds, their pharmaceutically acceptable salts, their solvates and polymorphs, defined in any aspect of the invention (except intermediate compounds in chemical processes) are referred to as "compounds of the invention".

The compounds of the invention may possess one or more chiral centres and so exist in a number of stereoisomeric forms. All stereoisomers and mixtures thereof are included in the scope of the present invention. Racemic compounds may either be separated using preparative HPLC and a column with a chiral stationary phase or resolved to yield individual enantiomers utilising methods known to those skilled in the art. In addition, chiral intermediate compounds may be resolved and used to prepare chiral compounds of the invention.

In cases where the compounds of the invention exist as the E and Z isomers, the invention includes individual isomers as well as mixtures thereof.

In cases where compounds of the invention exist as tautomeric isomers, the invention includes individual tautomers as well as mixtures thereof.

In cases where the compounds of the invention exist as optical isomers, the invention includes individual isomers as well as mixtures thereof.

In cases where the compounds of the invention exist as diastereoisomers, the invention includes individual diastereoisomers as well as mixtures thereof.

Separation of diastereoisomers or E and Z isomers may be achieved by conventional techniques, e.g. by fractional crystallisation, chromatography or H.P.L.C. An individual enantiomer of a compound of the invention or intermediate may be prepared from a corresponding optically pure intermediate or by resolution, such as by H.P.L.C. of the corresponding racemate using a suitable chiral support or by fractional crystallisation of the diastereoisomeric salts formed by reaction of the corresponding racemate with a suitable optically active base, as appropriate. A preferred optically active base is pseudoephedrine (see Preparation 7 herein).

The compounds of the invention may exist in one or more tautomeric forms. All tautomers and mixtures thereof are included in the scope of the present invention. For example, a claim to 2-hydroxypyridinyl would also cover its tautomeric form, α-pyridonyl.

It will be appreciated by those skilled in the art that certain protected derivatives of compounds of the invention, which may be made prior to a final deprotection stage, may not possess pharmacological activity as such, but may, in certain instances, be administered orally or parenterally and thereafter metabolised in the body to form compounds of the invention which are pharmacologically active. Such derivatives may therefore be described as "prodrugs". Further, certain compounds of the invention may act as prodrugs of other compounds of the invention.

All protected derivatives and prodrugs of compounds of the invention are included within the scope of the invention. Examples of suitable pro-drugs for the compounds of the present invention are described in Drugs of Today, Volume 19, Number 9, 1983, pp 499 - 538 and in Topics in Chemistry, Chapter 31, pp 306 - 316 and in "Design of Prodrugs" by H. Bundgaard, Elsevier, 1985, Chapter 1 (the disclosures in which documents are incorporated herein by reference).

It will further be appreciated by those skilled in the art, that certain moieties, known to those skilled in the art as "pro-moieties", for example as described by H. Bundgaard in "Design of Prodrugs" (the disclosure in which document is incorporated herein by reference) may be placed on appropriate functionalities when such functionalities are present within the compounds of the invention.

Preferred prodrugs for compounds of the invention include: esters, carbonate esters, hemi-esters, phosphate esters, nitro esters, sulfate esters, sulfoxides, amides, carbamates, azo-compounds, phosphamides, glycosides, ethers, acetals and ketals.

The invention also includes all suitable isotopic variations of the compounds of the invention. An isotopic variation is defined as one in which at least one atom is replaced by an atom having the same atomic number but an atomic mass different from the atomic mass usually found in nature. Examples of isotopes that can be incorporated into compounds of the invention include isotopes of hydrogen, carbon, nitrogen, oxygen, phosphorus, sulphur, fluorine and chlorine such as $^{2}H$, $^{3}H$, $^{13}C$, $^{14}C$, $^{15}N$, $^{17}O$, $^{18}O$, $^{31}P$, $^{32}P$, $^{35}S$, $^{18}F$ and $^{36}Cl$, respectively. Certain isotopic variations of the invention, for example, those in which a radioactive isotope such as $^{3}H$ or $^{14}C$ is incorporated, are useful in drug and/or substrate tissue distribution studies. Tritiated, i.e. $^{3}H$, and carbon-14, i.e. $^{14}C$ isotopes are particularly preferred for their ease of preparation and detectability. Further, substitution with isotopes such as deuterium, i.e. $^{2}H$, may afford certain therapeutic advantages resulting from greater metabolic stability, for example, increased *in vivo* half-life or reduced dosage requirements and hence may be preferred in some circumstances. Isotopic variations of the compounds of the invention can generally be prepared by conventional procedures such as by the methods or preparations described in the Examples and Preparations hereafter using appropriate isotopic variations of suitable reagents.

The compounds of the invention are inhibitors of the zinc-dependent, neutral endopeptidase EC.3.4.24.11., and it is proposed that the compounds of the invention will treat the disease states listed below. This enzyme is involved in the breakdown of several bioactive oligopeptides, cleaving peptide bonds on the amino side of hydrophobic amino acid residues. The peptides metabolised include atrial natriuretic peptides (ANP), bombesin, bradykinin, calcitonin gene-related peptide, endothelins, enkephalins, neurotensin, substance P and vasoactive intestinal peptide. Some of these peptides have potent vasodilatory and neurohormone functions, diuretic and natriuretic activity or mediate behaviour effects. Thus, the compounds of the invention, by inhibiting the neutral endopeptidase EC.3.4.24.11, can potentiate the biological effects of bioactive peptides.

Thus, in particular the compounds have utility in the treatment of a number of disorders, including hypertension, heart failure, angina, renal insufficiency, acute renal failure, cyclical oedema, Menières disease, hyperaldosteroneism (primary and secondary) and hypercalciuria. In addition, because of their ability to potentiate the effects of atrial natriuretic factor (ANF) the compounds have utility in the treatment of glaucoma. As a further result of their ability to inhibit the neutral endopeptidase E.C.3.4.24.11 the compounds of the invention may have activity in other therapeutic areas including for example the treatment of menstrual disorders, preterm labour, pre-eclampsia, endometriosis, and reproductive disorders (especially male and female infertility, polycystic ovarian syndrome, implantation failure). Also the compounds of the invention should treat asthma, inflammation, leukemia, pain, epilepsy, affective disorders, dementia and geriatric confusion, obesity and gastrointestinal disorders (especially diarrhoea and irritable bowel syndrome), wound healing (especially diabetic and venous ulcers and pressure sores), septic shock, the modulation of gastric acid secretion, the treatment of hyperreninaemia, cystic fibrosis, restenosis, diabetic complications and athereosclerosis.

The compounds of the invention are particularly beneficial for the treatment of female sexual dysfunction (FSD) (especially female sexual arousal disorder (FSAD)) and male sexual dysfunction (especially male erectile dysfunction (MED)).

Accordingly the present invention provides a compound of formula (I) as disclosed herein or pharmaceutically acceptable salts, solvates or prodrugs thereof for use as a medicament.

Further it provides the use of a compound of formula (I) as disclosed herein or pharmaceutically acceptable salts, solvates or prodrugs thereof in the preparation of a medicament for the prevention or treatment of a condition for which a beneficial response is obtained by the inhibition of neutral endopeptidase.

Also provided is a method of treating or preventing a condition in a mammal for which a beneficial response is obtained by the inhibition of neutral endopeptidase which comprises administering a therapeutically effective amount of a compound of formula (I) as disclosed herein or pharmaceutically acceptable salts, solvates or prodrugs thereof to a patient in need of such treatment.

Conditions capable of being so treated include: hypertension, heart failure, angina, renal insufficiency, acute renal failure, cyclical oedema, Menières disease, hyperaldosteroneism (primary and secondary), hypercalciuria, glaucoma, menstrual disorders, preterm labour, pre-eclampsia, endometriosis, reproductive disorders, asthma, inflammation, leukemia, pain, epilepsy, affective disorders, dementia and geriatric confusion, obesity, gastrointestinal disorders, wound healing, septic shock, the modulation of gastric acid secretion, the treatment of hyperreninaemia, cystic fibrosis, restenosis, diabetic complications, athereosclerosis, female sexual dysfunction (FSD) and male sexual dysfunction.

Particularly suitable conditions fortreatment are female sexual dysfunction and male sexual dysfunction; most particularly female sexual arousal dysfunction.

In accordance with the invention, FSD can be defined as the difficulty or inability of a woman to find satisfaction in sexual expression. FSD is a collective term for several diverse female sexual disorders (Leiblum, S.R. (1998). Definition and classification of female sexual disorders. *Int. J. Impotence Res.,* 10, S104-S106;, Berman, J.R., Berman, L. & Goldstein, I. (1999). Female sexual dysfunction: Incidence, pathophysiology, evaluations and treatment options. *Urology,* 54, 385-391). The woman may have lack of desire, difficulty with arousal or orgasm, pain with intercourse or a combination of these problems. Several types of disease, medications, injuries or psychological problems can cause FSD. Treatments in development are targeted to treat specific subtypes of FSD, predominantly desire and arousal disorders.

The categories of FSD are best defined by contrasting them to the phases of normal female sexual response: desire, arousal and orgasm (Leiblum, S.R. (1998). Definition and classification of female sexual disorders, *Int. J. Impotence Res.,* 10, S104-S106). Desire or libido is the drive for sexual expression. Its manifestations often include sexual thoughts either when in the company of an interested partner or when exposed to other erotic stimuli. Arousal is the vascular response to sexual stimulation, an important component of which is genital engorgement and includes increased vaginal lubrication, elongation of the vagina and increased genital sensation/sensitivity. Orgasm is the release of sexual tension that has culminated during arousal.

Hence, FSD occurs when a woman has an inadequate or unsatisfactory response in any of these phases, usually desire, arousal or orgasm. FSD categories include hypoactive sexual desire disorder, sexual arousal disorder, orgasmic disorders and sexual pain disorders. Although the compounds of the invention will improve the genital response to sexual stimulation (as in female sexual arousal disorder), in doing so it may also improve the associated pain, distress and discomfort associated with intercourse and so treat other female sexual disorders.

Hypoactive sexual desire disorder is present if a woman has no or little desire to be sexual, and has no or few sexual thoughts or fantasies. This type of FSD can be caused by low testosterone levels, due either to natural menopause or to surgical menopause. Other causes include illness, medications, fatigue, depression and anxiety.

Female sexual arousal disorder (FSAD) is characterised by inadequate genital response to sexual stimulation. The genitalia do not undergo the engorgement that characterises normal sexual arousal. The vaginal walls are poorly lubricated, so that intercourse is painful. Orgasms may be impeded. Arousal disorder can be caused by reduced oestrogen at menopause or after childbirth and during lactation, as well as by illnesses, with vascular components such as diabetes and atherosclerosis. Other causes result from treatment with diuretics, antihistamines, antidepressants eg SSRIs or antihypertensive agents.

Sexual pain disorders (includes dyspareunia and vaginismus) is characterised by pain resulting from penetration and may be caused by medications which reduce lubrication, endometriosis, pelvic inflammatory disease, inflammatory bowel disease or urinary tract problems.

The prevalence of FSD is difficult to gauge because the term covers several types of problem, some of which are difficult to measure, and because the interest in treating FSD is relatively recent. Many women's sexual problems are associated either directly with the female ageing process or with chronic illnesses such as diabetes and hypertension.

Because FSD consists of several subtypes that express symptoms in separate phases of the sexual response cycle, there is not a single therapy. Current treatment of FSD focuses principally on psychological or relationship issues. Treatment of FSD is gradually evolving as more clinical and basic science studies are dedicated to the investigation of this medical problem. Female sexual complaints are not all psychological in pathophysiology, especially for those individuals who may have a component of vasculogenic dysfunction (eg FSAD) contributing to the overall female sexual complaint. There are at present no drugs licensed for the treatment of FSD. Empirical drug therapy includes oestrogen administration (topically or as hormone replacement therapy), androgens or mood-altering drugs such as buspirone or trazodone. These treatment options are often unsatisfactory due to low efficacy or unacceptable side effects.

Since interest is relatively recent in treating FSD pharmacologically, therapy consists of the following:- psychological counselling, over-the-counter sexual lubricants, and investigational candidates, including drugs approved for other conditions. These medications consist of hormonal agents, either testosterone or combinations of oestrogen and testosterone and more recently vascular drugs, that have proved effective in male erectile dysfunction. None of these agents has been demonstrated to be very effective in treating FSD.

As discussed, the compounds of the invention are particularly useful for the treatment of female sexual arousal disorder (FSAD).

The Diagnostic and Statistical Manual (DSM) IV of the American Psychiatric Association defines Female Sexual Arousal Disorder (FSAD) as being:

> "a persistent or recurrent inability to attain or to maintain until completion of the sexual activity adequate lubrication-swelling response of sexual excitement. The disturbance must cause marked distress or interpersonal difficulty."

The arousal response consists of vasocongestion in the pelvis, vaginal lubrication and expansion and swelling of the external genitalia. The disturbance causes marked distress and/or interpersonal difficulty.

FSAD is a highly prevalent sexual disorder affecting pre-, peri- and post menopausal (±HRT) women. It is associated with concomitant disorders such as depression, cardiovascular diseases, diabetes and UG disorders.

The primary consequences of FSAD are lack of engorgement/swelling, lack of lubrication and lack of pleasurable genital sensation. The secondary consequences of FSAD are reduced sexual desire, pain during intercourse and difficulty in achieving an orgasm.

It has recently been hypothesised that there is a vascular basis for at least a proportion of patients with symptoms of FSAD (Goldstein *et al.,* Int. J. Impot. Res., 10, S84-S90,1998) with animal data supporting this view (Park *et al.*, Int. J. Impot. Res., 9, 27-37, 1997).

Drug candidates for treating FSAD, which are under investigation for efficacy, are primarily erectile dysfunction therapies that promote circulation to the male genitalia. They consist of two types of formulation, oral or sublingual medications (Apomorphine, Phentolamine, phosphodiesterase type 5 (PDE5) inhibitors e.g. Sildenafil), and prostaglandin ($PGE_1$) that are injected or administered transurethrally in men, and topically to the genitalia in women.

The compounds of the invention are advantageous by providing a means for restoring a normal sexual arousal response - namely increased genital blood flow leading to vaginal, clitoral and labial engorgement. This will result in increased vaginal lubrication *via* plasma transudation, increased vaginal compliance and increased genital sensitivity. Hence, the compounds of the invention provide means to restore, or potentiate, the normal sexual arousal response.

Without being bound by theory, we believe that neuropeptides such as vasoactive intestinal peptide (VIP) are

major neurotransmitter candidates in the control of the female sexual arousal response, especially in the control of genital blood flow. VIP and other neuropeptides are degraded/ metabolised by NEP EC3.4.24.11. Thus, NEP inhibitors will potentiate the endogenous vasorelaxant effect of VIP released during arousal. This will lead to a treatment of FSAD, such as through enhanced genital blood flow and hence genital engorgement. We have shown that selective inhibitors of NEP EC 3.4.24.11 enhance pelvic nerve-stimulated and VIP-induced increases in vaginal and clitoral blood flow. In addition, selective NEP inhibitors enhance VIP and nerve-mediated relaxations of isolated vagina wall.

Thus the present invention is advantageous as it helps provide a means for restoring a normal sexual arousal response - namely increased genital blood flow leading to vaginal, clitoral and labial engorgement. This will result in increased vaginal lubrication *via* plasma transudation, increased vaginal compliance and increased vaginal sensitivity. Hence, the present invention provides a means to restore, or potentiate the normal sexual arousal response.

Male sexual dysfunction includes male erectile dysfunction, ejaculatory disorders such as premature ejaculation (PE), anorgasmia (inability to achieve orgasm) and desire disorders such as hypoactive sexual desire disorder (lack of interest in sex).

It is to be appreciated that all references herein to treatment include curative, palliative and prophylactic treatment.

The compounds of the invention find application in the following sub-populations of patients with FSD: the young, the elderly, pre-menopausal, peri-menopausal, post-menopausal women with or without hormone replacement therapy.

The compounds of the invention find application in patients with FSD arising from:-

i) Vasculogenic etiologies eg cardiovascular or atherosclerotic diseases, hypercholesterolemia, cigarette smoking, diabetes, hypertension, radiation and perineal trauma, traumatic injury to the iliohypogastric pudendal vacular system.
ii) Neurogenic etiologies such as spinal cord injuries or diseases of the central nervous system including multiple sclerosis, diabetes, Parkinsonism, cerebrovascular accidents, peripheral neuropathies, trauma or radical pelvic surgery.
iii) Hormonal/endocrine etiologies such as dysfunction of the hypothalamic/pituitary/gonadal axis, or dysfunction of the ovaries, dysfunction of the pancreas, surgical or medical castration, androgen deficiency, high circulating levels of prolactin eg hyperprolactinemia, natural menopause, premature ovarian failure, hyper and hypothyroidism.
iv) Psychogenic etiologies such as depression, obsessive compulsive disorder, anxiety disorder, postnatal depression/"Baby Blues", emotional and relational issues, performance anxiety, marital discord, dysfunctional attitudes, sexual phobias, religious inhibition or a traumatic past experiences.
v) Drug-induced sexual dysfunction resulting from therapy with selective serotonin reuptake inhibitors (SSRis) and other antidepressant therapies (tricyclics and major tranquillizers), anti-hypertensive therapies, sympatholytic drugs, chronic oral contraceptive pill therapy.

Compounds of the invention may be prepared, in known manner in a variety of ways. In the following reaction schemes and hereafter, unless otherwise stated $R^1$, n, X and Y are as defined in the first aspect. These processes form further aspects of the invention.

Throughout the specification, general formulae are designated by Roman numerals I, II, III, IV etc. Subsets of these general formulae are defined as Ia, Ib, Ic etc, .... IVa, IVb, IVc etc.

Compounds of general formula I may be prepared according to reaction scheme 1, by reacting a compound of formula II (where Prot is a suitable protecting group) with a primary amine of formula III to give a compound of formula IV. Deprotection gives compounds of formula I.

Scheme 1

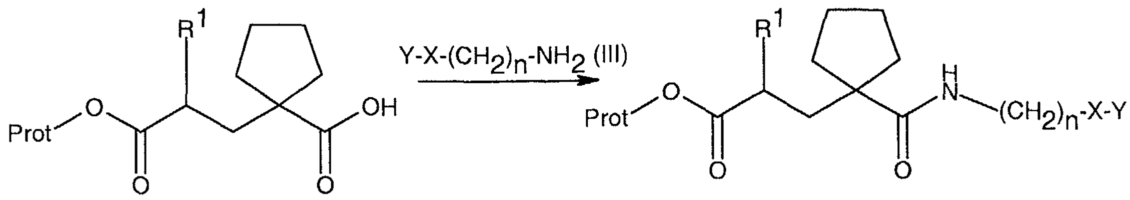


(II) (IV)

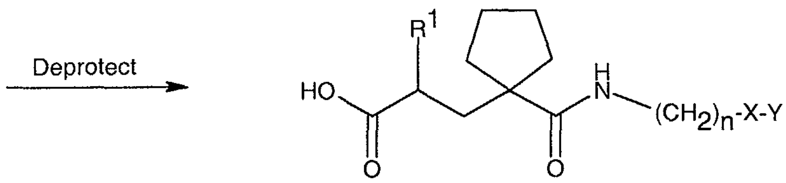


(I)

The acid/amine coupling step can be carried out by reacting compounds of formula II with compounds of formula III (or its amine salt) in the presence of a coupling agent, optionally a catalyst, and an excess of an acid acceptor, in a suitable solvent. Typically, treatment of a mixture of compounds of formula II and compounds of formula III with a coupling agent (for example dicyclohexylcarbodiimide (DCC), 1-(3-dimethylaminopropyl)-3-ethylcarbodiimide (WSCDI), benzotriazol-1-yl diethyl phosphate, phosphorus oxychloride, titanium tetrachloride, sulfuryl chloride fluoride, Lawesson's reagent, PPACA, PYBOP or Mukaiyama's reagent) optionally in the presence of a tertiary amine base (for example triethylamine, Hunig's base, pyridine or NMM) for up to 24 hours at temperatures between -78 and 100 °C. Preferred reaction conditions comprise reacting compounds of formula II (1-1.5 equivalents) with compounds of formula III (or their salts 1-1.5 equivalents), in the presence of 1-(3-dimethylaminopropyl)-3-ethylcarbodiimide hydrochloride (WSCDI) or N,N'-dicyclohexylcarbodiimide (DCC) (1.1-1.3 equivalents), 1-hydroxybenzotrazole hydrate (HOBT) or dimethylaminopyridine (DMAP) (1.05-1.2 equivalents), N-methyl morpholine (NMM) or triethyamine (2.3-3 equivalents) in dimethylformamide or dichloromethane at between room temperature and 90°C for 16-18 hours.

Alternatively, the acid/amine coupling step may proceed via an activated intermediate (such as an acyl imidazolide, mixed anhydride or acid chloride) in the presence of an excess of acid acceptor in a suitable solvent. Typical reaction conditions comprise treatment of compounds of formula II with an activating agent (for example *N,N'*-carbonyldiimidazole, *N,N'*-carbonylbis(3-methylimidazolium) triflate, thionyl chloride or oxalyl chloride) optionally in the presence of a tertiary amine base (for example triethylamine, Hunig's base, pyridine or NMM) for up to 24 hours followed by reaction with compounds of formula III (or its salt), optionally in the presence of a catalyst (for example 4-dimethylaminopyridine) or an additive (for example hydroxybenzotriazole) in a suitable solvent (for example dichloromethane, THF, ethyl acetate, acetonitrile, DMF or toluene) optionally in the presence of an additional amine base at temperatures between -78 °C and 150 °C for up to 48 hours.

Preferred reaction conditions comprise reacting the acid chloride of compounds of formula II (1-1.1 equivalents) with compounds of formula III (or their salts, 1 to 1.5 equivalents) in the presence of triethyamine or *N*-methyl morpholine (1.4-10 equivalents) in dichloromethane solvent at room temperature for 24 hours. Alternatively, compounds of formula II can be converted to the acid chloride *in situ* by treatment with oxalyl chloride in dichloromethane in the presence of a catalytic amount of dimethylformamide for 2 hours at room temperature or by treatment of compounds of formula II with thionyl chloride in a mixture of dichloromethane and pyridine at -10 °C for 3 hours followed by addition of triethylamine, 4-dimethylaminopyridine and the compound of formula III and allowing the mixture to react for 48 hours at 20 °C.

Compounds of formula I may be prepared from compounds of formula IV by deprotection. Methods for deprotection of an acid group depend on the protecting group. For examples of protection/deprotection methodology see "Protective groups in Organic synthesis", TW Greene and PGM Wutz.

For example, when Prot is a *tert*-butyl, deprotection conditions comprise reacting IV with trifluoroacetic acid/dichloromethane (1:1-1.5 by volume), at room temperature for 2-18 hours, optionally in the presence of a carbocation scavenger, e.g. anisole (10 equivalents). When Y contains a hydroxy group, base hydrolysis of the intermediate trifluoroacetic acid ester may be necessary. Alternative methodology for deprotection when Prot is *tert*-butyl comprises treating IV with hydrochloric acid in dichloromethane at room temperature for 3 hours. For the avoidance of doubt, Prot as *tert*-butyl is given by way of Example and is not intended to be limited to *tert*-Butyl.

Alternatively, when Prot is *tert*-butyl deprotection may be achieved by treating compounds of formula IV with a strong acid (for example gaseous or concentrated hydrochloric acid, hydrobromic acid, phosphoric acid, nitric acid or sulfuric acid, trifluoroacetic acid, chloroacetic acid, *para*-toluenesulfonic acid, trifluoromethanesulfonic acid or glacial acetic acid) in quantities ranging from catalytic to an excess, optionally in a suitable solvent (for example toluene, dichloromethane, diethyl ether, ethanol, THF or hexane) and optionally in the presence of water at temperatures between 20 °C and 150 °C for up to 48 hours.

Preferred deprotection conditions when Prot is *tert*-butyl are treatment of compounds of formula IV with a tenfold excess of trifluoroacetic acid in dichloromethane at room temperature for 24 hours.

When Prot is benzyl, deprotection conditions comprise reacting IV with palladium on charcoal (5-10%) in aqueous ethanol (40-95%) at 15-60 psi at room temperature for 2hrs to 3 days.

These processes form further aspects of the invention.

Compounds of formula IV are novel and form a further aspect of the invention.

All of the above reactions and the preparations of novel starting materials used in the preceding methods are conventional. Appropriate reagents and reaction conditions for their performance or preparation as well as procedures for isolating the desired products will be well-known to those skilled in the art with reference to literature precedents and the Examples and Preparations hereinbelow.

A pharmaceutically acceptable salt of a compound of the formula (I) may be readily prepared by mixing together solutions of a compound of the formula (I) and the desired acid or base, as appropriate. The salt may precipitate from solution and be collected by filtration or may be recovered by evaporation of the solvent.

The compounds of the invention may also be combined with one or more of the following for the treatment of FSD:

1) One or more naturally occurring or synthetic prostaglandins or esters thereof. Suitable prostaglandins for use herein include compounds such as alprostadil, prostaglandin $E_1$,prostaglandin $E_0$, 13, 14 - dihydroprosta glandin $E_1$, prostaglandin $E_2$, eprostinol, natural synthetic and semisynthetic prostaglandins and derivatives thereof including those described in WO-00033825 and/or US 6,037,346 issued on 14th March 2000 all incorporated herein by reference, $PGE_0$, $PGE_1$, $PGA_1$, $PGB_1$, $PGF_1$ α, 19-hydroxy $PGA_1$, 19-hydroxy - $PGB_1$, $PGE_2$, $PGB_2$, 19-hydroxy-$PGA_2$, 19-hydroxy-$PGB_2$, $PGE_3\alpha$, carboprost tromethamine dinoprost, tromethamine, dinoprostone, lipo prost, gemeprost, metenoprost, sulprostune, tiaprost and moxisylate.

2) One or more α-adrenergic receptor antagonist compounds also known as α-adrenoceptors or α-receptors or α-blockers. Suitable compounds for use herein include: the α-adrenergic receptor blockerss as described in PCT application WO99/30697 published on 14th June 1998, the disclosures of which relating to α-adrenergic receptors are incorporated herein by reference and include, selective $\alpha_1$-adrenoceptor or $\alpha_2$-adrenoceptor blockers and non-selective adrenoceptor blockers, suitable $\alpha_1$-adrenoceptor blockers include: phentolamine, phentolamine mesylate, trazodone, alfuzosin, indoramin, naftopidil, tamsulosin, dapiprazole, phenoxybenzamine, idazoxan, efaraxan, yohimbine, rauwolfa alkaloids, Recordati 15/2739, SNAP 1069, SNAP 5089, RS17053, SL 89.0591, doxazosin, terazosin, abanoquil and prazosin; $\alpha_2$-blocker blockers from US 6,037,346 [14th March 2000] dibenarnine, tolazoline, trimazosin and dibenarnine; α-adrenergic receptors as described in US patents: 4,188,390; 4,026,894; 3,511,836; 4,315,007; 3,527,761; 3,997,666; 2,503,059; 4,703,063; 3,381,009; 4,252,721 and 2,599,000 each of which is incorporated herein by reference; $\alpha_2$-Adrenoceptor blockers include: clonidine, papaverine, papaverine hydrochloride, optionally in the presence of a cariotonic agent such as pirxamine.

3) One or more NO-donor (NO-agonist) compounds. Suitable NO-donor compounds for use herein include organic nitrates, such as mono- di or tri-nitrates or organic nitrate esters including glyceryl brinitrate (also known as nitroglycerin), isosorbide 5-mononitrate, isosorbide dinitrate, pentaerythritol tetranitrate, erythrityl tetranitrate, sodium nitroprusside (SNP), 3-morpholinosydnonimine molsidomine, S-nitroso- N-acetyl penicilliamine (SNAP) S-nitroso-N-glutathione (SNO-GLU), N-hydroxy - L-arginine, amylnitrate, linsidomine, linsidomine chlorohydrate, (SIN-1) S-nitroso-N-cysteine, diazenium diolates, (NONOates), 1,5-pentanedinitrate, L-arginene, ginseng, zizphi fructus, molsidomine, Re-2047, nitrosylated maxisylyte derivatives such as NMI-678-11 and NMI-937 as described in published PCT application WO 0012075.

4) One or more potassium channel openers or modulators. Suitable potassium channel openers/modulators for use herein include nicorandil, cromokalim, levcromakalim, lemakalim, pinacidil, cliazoxide, minoxidil, charybdotoxin, glyburide, 4-amini pyridine, $BaCl_2$.

5) One or more dopaminergic agents, preferably apomorphine or a selective D2, D3 or D2/$D_3$agonist such as,

pramipexole and ropirinol (as claimed in WO-0023056), PNU95666 (as claimed in WO-0040226).

6) One or more vasodilator agents. Suitable vasodilator agents for use herein include nimodepine, pinacidil, cyclandelate, isoxsuprine, chloroprumazine, halo peridol, Rec 15/2739, trazodone.

7) One or more thromboxane A2 agonists.

8) One or more CNS active agents.

9) One or more ergot alkoloids. Suitable ergot alkaloids are described in US patent 6,037,346 issued on 14th March 2000 and include acetergamine, brazergoline, bromerguride, cianergoline, delorgotrile, disulergine, ergonovine maleate, ergotamine tartrate, etisulergine, lergotrile, lysergide, mesulergine, metergoline, metergotamine, nicergoline, pergolide, propisergide, proterguride, terguride.

10) One or more compounds which modulate the action of natruretic factors in particular atrial naturetic factor (also known as atrial naturetic peptide), B type and C type naturetic factors such as inhibitors or neutral endopeptidase.

11) One or more compounds which inhibit angiotensin-converting enzyme such as enapril, and combined inhibitors of angiotensin-converting enzyme and neutral endopeptidase such as omapatrilat.

12) One or more angiotensin receptor antagonists such as losartan.

13) One or more substrates for NO-synthase, such as L-arginine.

14) One or more calcium channel blockers such as amlodipine.

15) One or more antagonists of endothelin receptors and inhibitors or endothelin-converting enzyme.

16) One or more cholesterol lowering agents such as statins (e.g. atorvastatin/Lipitor- trade mark) and fibrates.

17) One or more antiplatelet and antithrombotic agents, e.g. tPA, uPA, warfarin, hirudin and other thrombin inhibitors, heparin, thromboplastin activating factor inhibitors.

18) One or more insulin sensitising agents such as rezulin and hypoglycaemic agents such as glipizide.

19) L-DOPA or carbidopa.

20) One or more acetylcholinesterase inhibitors such as donezipil.

21) One or more steroidal or non-steroidal anti-inflammatory agents.

22) One or more estrogen receptor modulators and/or estrogen agonists and/or estrogen antagonists, preferably raloxifene or lasofoxifene, (-)-cis-6-phenyl-5-[4-(2-pyrrolidin-1-yl-ethoxy)-phenyl]-5,6,7,8-tetrahydronaphthalene-2-ol and pharmaceutically acceptable salts thereof the preparation of which is detailed in WO 96/21656.

23) One or more modulators of cannabinoid receptors.

24) One or more of an NPY (neuropeptide Y) inhibitor, more particularly NPY1 or NPY5 inhibitor, preferably NPY1 inhibitor, preferably said NPY inhibitors (including NPY Y1 and NPY Y5) having an IC50 of less than 100nM , more preferably less than 50nM. An assay for identifying NPY inhibitors is presented in WO-A-98/52890 (see page 96, lines 2 to 28).

25) One or more of vasoactive intestinal protein (VIP), VIP mimetic, VIP analogue, more particularly mediated by one or more of the VIP receptor subtypes VPAC1,VPAC or PACAP (pituitory adenylate cyclase activating peptide), one or more of a VIP receptor agonist or a VIP analogue (eg Ro-125-1553) or a VIP fragment, one or more of a α-adrenoceptor antagonist with VIP combination (eg Invicorp, Aviptadil).

26) One or more of a melanocortin receptor agonist or modulator or melanocortin ehancer, such as melanotan II, PT-14, PT-141 or compounds claimed in WO-09964002, WO-00074679, WO-09955679, WO-00105401, WO-00058361, WO-00114879, WO-00113112, WO-09954358.

27) One or more of a serotonin receptor agonist, antagonist or modulator, more particularly agonists, antagonists or modulators for 5HT1A (including VML 670), 5HT2A, 5HT2C, 5HT3 and/or 5HT6 receptors, including those described in WO-09902159, WO-00002550 and/or WO-00028993.

28) One or more of a testosterone replacement agent (inc dehydroandrostendione), testosternone (Tostrelle), dihydrotestosterone or a testosterone implant.

29) One or more of estrogen, estrogen and medroxyprogesterone or medroxyprogesterone acetate (MPA) (i.e. as a combination), or estrogen and methyl testosterone hormone replacement therapy agent (e.g. HRT especially Premarin, Cenestin, Oestrofeminal, Equin, Estrace, Estrofem, Elleste Solo, Estring, Eastraderm TTS, Eastraderm Matrix, Dermestril, Premphase, Preempro, Prempak, Premique, Estratest, Estratest HS, Tibolone).

30) One or more of a modulator of transporters for noradrenaline, dopamine and/or serotonin, such as bupropion, GW-320659.

31) One or more of a purinergic receptor agonist and/or modulator.

32) One or more of a neurokinin (NK) receptor antagonist, including those described in WO-09964008.

33) One or more of an opioid receptor agonist, antagonist or modulator, preferably agonists for the ORL-1 receptor.

34) One or more of an agonist or modulator for oxytocin/vasopressin receptors, preferably a selective oxytocin agonist or modulator.

35) One or more of a PDE inhibitor, more particularly a PDE 2, 3, 4, 5, 7 or 8 inhibitor, preferably PDE2 or PDE5 inhibitor and most preferably a PDE5 inhibitor (see hereinafter), said inhibitors preferably having an IC50 against the respective enzyme of less than 100nM. Suitable cGMP PDE5 inhibitors for the use according to the present

invention include:

the pyrazolo [4,3-d]pyrimidin-7-ones disclosed in EP-A-0463756; the pyrazolo [4,3-d]pyrimidin-7-ones disclosed in EP-A-0526004; the pyrazolo [4,3-d]pyrimidin-7-ones disclosed in published international patent application WO 93/06104; the isomeric pyrazolo [3,4-d]pyrimidin-4-ones disclosed in published international patent application WO 93/07149; the quinazolin-4-ones disclosed in published international patent application WO 93/12095; the pyrido [3,2-d]pyrimidin-4-ones disclosed in published international patent application WO 94/05661; the purin-6-ones disclosed in published international patent application WO 94/00453; the pyrazolo [4,3-d]pyrimidin-7-ones disclosed in published international patent application WO 98/49166; the pyrazolo [4,3-d]pyrimidin-7-ones disclosed in published international patent application WO 99/54333; the pyrazolo [4,3-d]pyrimidin-4-ones disclosed in EP-A-0995751; the pyrazolo [4,3-d]pyrimidin-7-ones disclosed in published international patent application WO 00/24745; the pyrazolo [4,3-d]pyrimidin-4-ones disclosed in EP-A-0995750; the compounds disclosed in published international application WO95/19978; the compounds disclosed in published international application WO 99/24433 and the compounds disclosed in published international application WO 93/07124. The pyrazolo [4,3-d]pyrimidin-7-ones disclosed in published international application WO 01/27112; the pyrazolo [4,3-d]pyrimidin-7-ones disclosed in published international application WO 01/27113; the compounds disclosed in EP-A-1092718 and the compounds disclose din EP-A-1092719.

Further suitable PDE5 inhibitors for the use according to the present invention include: 5-[2-ethoxy-5-(4-methyl-1-piperazinylsulphonyl)phenyl]-1-methyl-3-n-propyl-1,6-dihydro-7H-pyrazolo[4,3-d]pyrimidin-7-one (sildenafil) also known as 1-[[3-(6,7-dihydro-1-methyl-7-oxo-3-propyl-1H-pyrazolo[4,3-d]pyrimidin-5-yl)-4-ethoxyphenyl]sulphonyl]-4-methylpiperazine (see EP-A-0463756); 5-(2-ethoxy-5-morpholinoacetylphenyl)-1-methyl-3-n-propyl-1,6-dihydro-7H-pyrazolo[4,3-d]pyrimidin-7-one(seeEP-A-0526004);3-ethyl-5-[5-(4-ethylpiperazin-1-ylsulphonyl)-2-n-propoxyphenyl]-2-(pyridin-2-yl)methyl-2,6-dihydro-7H-pyrazolo[4,3-d]pyrimidin-7-one (see WO98/49166); 3-ethyl-5-[5-(4-ethylpiperazin-1-ylsulphonyl)-2-(2-methoxyethoxy)pyridin-3-yl]-2-(pyridin-2-yl)methyl-2,6-dihydro-7H-pyrazolo[4,3-d]pyrimidin-7-one (see WO99/54333); (+)-3-ethyl-5-[5-(4-ethylpiperazin-1-ylsulphonyl)-2-(2-methoxy-1 (R)-methylethoxy)pyridin-3-yl]-2-methyl-2,6-dihydro-7H-pyrazolo[4,3-d]pyrimidin-7-one, also known as 3-ethyl-5-{5-[4-ethylpiperazin-1-ylsulphonyl]-2-([(1 R)-2-methoxy-1-methylethyl]oxy)pyridin-3-yl}-2-methyl-2,6-dihydro-7H-pyrazolo[4,3-d] pyrimidin-7-one (see WO99/54333); 5-[2-ethoxy-5-(4-ethylpiperazin-1-ylsulphonyl)pyridin-3-yl]-3-ethyl-2-[2-methoxyethyl]-2,6-dihydro-7H-pyrazolo[4,3-d]pyrimidin-7-one, also known as 1-{6-ethoxy-5-[3-ethyl-6,7-dihydro-2-(2-methoxyethyl)-7-oxo-2H-pyrazolo[4,3-d]pyrimidin-5-yl]-3-pyridylsulphonyl}-4-ethylpiperazine (see WO 01/27113, Example 8); 5-[2-*iso*-Butoxy-5-(4-ethylpiperazin-1-ylsulphonyl)pyridin-3-yl]-3-ethyl-2-(1-methylpiperidin-4-yl)-2,6-dihydro-7H-pyrazolo[4,3-d]pyrimidin-7-one (see WO 01/27113, Example 15); 5-[2-Ethoxy-5-(4-ethylpiperazin-1-ylsulphonyl)pyridin-3-yl]-3-ethyl-2-phenyl-2,6-dihydro-7H-pyrazolo[4,3-d]pyrimidin-7-one (see WO 01/27113, Example 66); 5-(5-Acetyl-2-propoxy-3-pyridinyl)-3-ethyl-2-(1-isopropyl-3-azetidinyl)-2,6-dihydro-7*H*-pyrazolo[4,3-*d*]pyrimidin-7-one (seeWO 01/27112, Example 124); 5-(5-Acetyl-2-butoxy-3-pyridinyl)-3-ethyl-2-(1-ethyl-3-azetidinyl)-2,6-dihydro-7*H*-pyrazolo[4,3-*d*]pyrimidin-7-one (see WO 01/27112, Example 132); (6R,12aR)-2,3,6,7,12,12a-hexahydro-2-methyl-6-(3,4-methylenedioxyphenyl) -pyrazino[2',1':6,1]pyrido [3,4-b]indole-1,4-dione (IC-351), i.e. the compound of examples 78 and 95 of published international application WO95/19978, as well as the compound of examples 1, 3, 7 and 8; 2-[2-ethoxy-5-(4-ethyl-piperazin-1-yl-1-sulphonyl)-phenyl]-5-methyl-7-propyl-3H-imidazo[5,1-f][1,2,4]triazin-4-one (vardenafil) also known as 1-[[3-(3,4-dihydro-5-methyl-4-oxo-7-propylimidazo[5,1-f]-as-triazin-2-yl)-4-ethoxyphenyl]sulphonyl]-4-ethylpiperazine, i.e. the compound of examples 20, 19, 337 and 336 of published international application WO99/24433; and the compound of example 11 of published international application WO93/07124 (EISAI); and compounds 3 and 14 from Rotella D P, *J. Med. Chem.,* 2000, 43, 1257. Still other suitable PDE5 inhibitors include:4-bromo-5-(pyridylmethylamino)-6-[3-(4-chlorophenyl)-propoxy]-3(2H)pyridazinone;1-[4-[(1,3-benzodioxol-5- ylmethyl)amiono]-6-chloro-2-quinozolinyl]-4-piperidine-carboxylic acid, monosodium salt; (+)-cis-5,6a,7,9,9,9a-hexahydro-2-[4-(trifluoromethyl)-phenylmethyl-5-methyl-cyclopent-4,5]imidazo[2,1-b]purin-4 (3H)one; furazlocillin; cis-2-hexyl-5-methyl-3,4,5,6a,7,8,9,9a- octahydrocyclopent[4,5]-imidazo[2,1-b]purin-4-one; 3-acetyl-1-(2-chlorobenzyl)-2-propylindole-6- carboxylate; 3-acetyl-1-(2-chlorobenzyl)-2-propylindole-6-carboxylate; 4-bromo-5-(3-pyridylmethylamino)-6-(3-(4-chlorophenyl) propoxy)-3- (2H)pyridazinone; I-methyl-5(5-morpholinoacetyl-2-n-propoxyphenyl)-3-n-propyl-1,6-dihydro-7H-pyrazolo(4,3-d)pyrimidin-7-one; 1-[4-[(1,3-benzodioxol-5-ylmethyl)arnino]-6-chloro-2- quinazolinyl]-4-piperidinecarboxylic acid, monosodium salt; Pharmaprojects No. 4516 (Glaxo Wellcome); Pharmaprojects No. 5051 (Bayer); Pharmaprojects No. 5064 (Kyowa Hakko; see WO 96/26940); Pharmaprojects No. 5069 (Schering Plough); GF-196960 (Glaxo Wellcome); E-8010 and E-4010 (Eisai); Bay-38-3045 & 38-9456 (Bayer) and Sch-51866.

If a combination of active agents are administered, then they may be administered simultaneously, separately

or sequentially.

The compounds of the invention can be administered alone but, in human therapy will generally be administered in admixture with a suitable pharmaceutical excipient diluent or carrier selected with regard to the intended route of administration and standard pharmaceutical practice.

Accordingly the present invention provides for a pharmaceutical composition comprising a compound of formula (I) as disclosed herein or pharmaceutically acceptable salts, solvates or prodrugs thereof; and a pharmaceutically acceptable diluent or carrier.

For example, the compounds of the invention, can be administered orally, buccally or sublingually in the form of tablets, capsules (including soft gel capsules), ovules, elixirs, solutions or suspensions, which may contain flavouring or colouring agents, for immediate-, delayed-, modified-, sustained-, dual-, controlled-release or pulsatile delivery applications. The compounds of the invention may also be administered via fast dispersing or fast dissolving dosage forms.

Modified release and pulsatile release dosage forms may contain excipients such as those detailed for immediate release dosage forms together with additional excipients that act as release rate modifiers, these being coated on and/or included in the body of the device. Release rate modifiers include, but are not exclusively limited to, hydroxypropylmethyl cellulose, methyl cellulose, sodium carboxymethylcellulose, ethyl cellulose, cellulose acetate, polyethylene oxide, Xanthan gum, Carbomer, ammonio methacrylate copolymer, hydrogenated castor oil, carnauba wax, paraffin wax, cellulose acetate phthalate, hydroxypropylmethyl cellulose phthalate, methacrylic acid copolymer and mixtures thereof. Modified release and pulsatile release dosage forms may contain one or a combination of release rate modifying excipients. Release rate modifying excipients may be present both within the dosage form i.e. within the matrix, and/or on the dosage form, i.e. upon the surface or coating.

Fast dispersing or dissolving dosage formulations (FDDFs) may contain the following ingredients: aspartame, acesulfame potassium, citric acid, croscarmellose sodium, crospovidone, diascorbic acid, ethyl acrylate, ethyl cellulose, gelatin, hydroxypropylmethyl cellulose, magnesium stearate, mannitol, methyl methacrylate, mint flavouring, polyethylene glycol, fumed silica, silicon dioxide, sodium starch glycolate, sodium stearyl fumarate, sorbitol, xylitol. The terms dispersing or dissolving as used herein to describe FDDFs are dependent upon the solubility of the drug substance used, i.e. where the drug substance is insoluble a fast dispersing dosage form can be prepared and where the drug substance is soluble a fast dissolving dosage form can be prepared.

The compositions of the invention may be administered by direct injection. The composition may be formulated for parenteral, mucosal, intramuscular, intravenous, subcutaneous, ocular, intraocular or transdermal administration. Depending upon the need, the agent may be administered at a dose of from 0.01 to 30 mg/kg body weight, such as from 0.1 to 10 mg/kg, more preferably from 0.1 to 1 mg/kg body weight.

The term "administered" includes delivery by viral or non-viral techniques. Viral delivery mechanisms include but are not limited to adenoviral vectors, adeno-associated viral (AAV) vectos, herpes viral vectors, retroviral vectors, lentiviral vectors, and baculoviral vectors. Non-viral delivery mechanisms include lipid mediated transfection, liposomes, immunoliposomes, lipofectin, cationic facial amphiphiles (CFAs) and combinations thereof. The routes for such delivery mechanisms include but are not limited to mucosal, nasal, oral, parenteral, gastrointestinal, topical, or sublingual routes.

In addition or in the alternative the compositions (or component parts thereof) of the present invention may be administered by direct injection. In addition or in the alternative the compositions (or component parts thereof) of the present invention may be administered topically (preferably to the genitalia). In addition or in the alternative the compositions (or component parts thereof) of the present invention may be administered by inhalation. In addition or in the alternative the compositions (or component parts thereof) of the present invention may also be administered by one or more of: a mucosal route, for example, as a nasal spray or aerosol for inhalation or as an ingestable solution such as by an oral route, or by a parenteral route where delivery is by an injectable form, such as, for example, by a rectal, ophthalmic (including intravitreal or intracameral), nasal, topical (including buccal and sublingual), intrauterine, vaginal or parenteral (including subcutaneous, intraperitoneal, intramuscular, intravenous, intradermal, intracranial, intratracheal, and epidural) transdermal, intraperitoneal, intracranial, intracerebroventricular, intracerebral, intravaginal, intrauterine, or parenteral (e.g., intravenous, intraspinal, subcutaneous, transdermal or intramuscular) route.

By way of example, the pharmaceutical compositions of the invention may be administered in accordance with a regimen of 1 to 10 times per day, such as once or twice per day. The specific dose level and frequency of dosage for any particular patient may be varied and will depend upon a variety of factors including the activity of the specific compound employed, the metabolic stability and length of action of that compound, the age, body weight, general health, sex, diet, mode and time of administration, rate of excretion, drug combination, the severity of the particular condition, and the individual undergoing therapy.

Hence, the term "administered" includes but is not limited to delivery by a mucosal route, for example, as a nasal spray or aerosol for inhalation or as an ingestable solution; a parenteral route where delivery is by an injectable form, such as, for example, an intravenous, intramuscular or subcutaneous route.

Such tablets may contain excipients such as microcrystalline cellulose, lactose, sodium citrate, calcium carbonate, dibasic calcium phosphate and glycine, disintegrants such as starch (preferably corn, potato or tapioca starch), sodium starch glycollate, croscarmellose sodium and certain complex silicates, and granulation binders such as polyvinylpyrrolidone, hydroxypropylmethyl cellulose (HPMC), hydroxypropylcellulose (HPC), sucrose, gelatin and acacia. Additionally, lubricating agents such as magnesium stearate, stearic acid, glyceryl behenate and talc may be included.

Solid compositions of a similar type may also be employed as fillers in gelatin capsules. Preferred excipients in this regard include lactose, starch, a cellulose, milk sugar or high molecular weight polyethylene glycols. For aqueous suspensions and/or elixirs, the compounds of the invention may be combined with various sweetening or flavouring agents, colouring matter or dyes, with emulsifying and/or suspending agents and with diluents such as water, ethanol, propylene glycol and glycerin, and combinations thereof.

The compounds of the invention can also be administered parenterally, for example, intravenously, intraarterially, intraperitoneally, intrathecally, intraventricularly, intraurethrally intrasternally, intracranially, intramuscularly or subcutaneously, or they may be administered by infusion techniques. In addition, they may be administered in the form of an implant. For such parenteral administration they are best used in the form of a sterile aqueous solution which may contain other substances, for example, enough salts or glucose to make the solution isotonic with blood. The aqueous solutions should be suitably buffered (preferably to a pH of from 3 to 9), if necessary. The preparation of suitable parenteral formulations under sterile conditions is readily accomplished by standard pharmaceutical techniques well-known to those skilled in the art. Parenteral formulations may be formulated for immediate-, delayed-, modified-, sustained-, dual-, controlled-release or pulsatile delivery.

The following dosage levels and other dosage levels herein are for the average human subject having a weight range of about 65 to 70 kg. The skilled person will readily be able to determine the dosage levels required for a subject whose weight falls outside this range, such as children and the elderly.

For oral and parenteral administration to human patients, the daily dosage level of the compounds of the invention or salts or solvates thereof will usually be from 10 to 1000 mg (in single or divided doses).

Thus, for example, tablets or capsules of the compounds of the invention or salts or solvates thereof may contain from 5 to 1000mg, such as 5mg to 500 mg of active compound for administration singly or two or more at a time, as appropriate. The physician in any event will determine the actual dosage which will be most suitable for any individual patient and it will vary with the age, weight and response of the particular patient. The above dosages are exemplary of the average case. There can, of course, be individual instances where higher or lower dosage ranges are merited and such are within the scope of this invention. The skilled person will also appreciate that, in the treatment of certain conditions (including FSD), compounds of the invention may be taken as a single dose on an "as required" basis (i.e. as needed or desired).

The compounds of the invention can also be administered intranasally or by inhalation and are conveniently delivered in the form of a dry powder inhaler or an aerosol spray presentation from a pressurised container, pump, spray or nebuliser with the use of a suitable propellant, e.g. dichlorodifluoromethane, trichlorofluoromethane, dichlorotetrafluoroethane, a hydrofluoroalkane such as 1,1,1,2-tetrafluoroethane (HFA 134A [trade mark] or 1,1,1,2,3,3,3-heptafluoropropane (HFA 227EA [trade mark]), carbon dioxide or other suitable gas. In the case of a pressurised aerosol, the dosage unit may be determined by providing a valve to deliver a metered amount. The pressurised container, pump, spray or nebuliser may contain a solution or suspension of the active compound, e.g. using a mixture of ethanol and the propellant as the solvent, which may additionally contain a lubricant, e.g. sorbitan trioleate. Capsules and cartridges (made, for example, from gelatin) for use in an inhaler or insufflator may be formulated to contain a powder mix of a compound of the invention and a suitable powder base such as lactose or starch.

Aerosol or dry powder formulations are preferably arranged so that each metered dose or "puff" contains from 1 to 50 mg of a compound of the invention for delivery to the patient. The overall daily dose with an aerosol will be in the range of from 1 to 50 mg which may be administered in a single dose or, more usually, in divided doses throughout the day.

Alternatively, compounds of the invention can be administered in the form of a suppository or pessary, or they may be applied topically (preferably to the genitalia) in the form of a gel, hydrogel, lotion, solution, cream, ointment or dusting powder. The compounds of the invention may also be dermally administered. The compounds of the invention may also be transdermally administered, for example, by the use of a skin patch. They may also be administered by the ocular, pulmonary or rectal routes.

For ophthalmic use, compounds can be formulated as micronised suspensions in isotonic, pH adjusted, sterile saline, or, preferably, as solutions in isotonic, pH adjusted, sterile saline, optionally in combination with a preservative such as a benzylalkonium chloride. Alternatively, they may be formulated in an ointment such as petrolatum.

For application topically to the skin (preferably to the genitalia), compounds of the invention can be formulated as a suitable ointment containing the active compound suspended or dissolved in, for example, a mixture with one or more of the following: mineral oil, liquid petrolatum, white petrolatum, propylene glycol, polyoxyethylene polyoxypropylene compound, emulsifying wax and water. Alternatively, they can be formulated as a suitable lotion or cream, suspended or dissolved in, for example, a mixture of one or more of the following: mineral oil, sorbitan monostearate,

a polyethylene glycol, liquid paraffin, polysorbate 60, cetyl esters wax, cetearyl alcohol, 2-octyldodecanol, benzyl alcohol and water.

The compounds of the invention may also be used in combination with a cyclodextrin. Cyclodextrins are known to form inclusion and non-inclusion complexes with drug molecules. Formation of a drug-cyclodextrin complex may modify the solubility, dissolution rate, bioavailability and/or stability property of a drug molecule. Drug-cyclodextrin complexes are generally useful for most dosage forms and administration routes. As an alternative to direct complexation with the drug the cyclodextrin may be used as an auxiliary additive, e.g. as a carrier, diluent or solubiliser. Alpha-, beta- and gamma-cyclodextrins are most commonly used and suitable examples are described in WO-A-91/11172, WO-A-94/02518 and WO-A-98/55148.

In a preferred embodiment, the compounds of the invention are delivered systemically (such as orally, buccally and sublingually), more preferably orally.

Preferably such systemic (most preferably oral) administration is used to treat female sexual dysfunction, preferably FSAD.

Thus in a particularly preferred embodiment, there is provided the use of the compounds of the invention in the manufacture of a systemically delivered (preferably orally delivered) medicament for the treatment or prophylaxis of FSD, more preferably FSAD.

A preferred oral formulation uses immediate release tablets; or fast dispersing or dissolving dosage formulations (FDDFs).

In a further preferred embodiment, the compounds of the invention are administered topically, preferably directly to the female genitalia, especially the vagina.

Since NEP is present throughout the body, it is very unexpected that the compounds of the invention can be administered systemically and achieve a therapeutic response in the female genitalia without provoking intolerable (adverse) side effects. In EP1097719-A1, we have shown that NEP inhibitors administered to a rabbit model *(in vivo)* systemically increased genital blood flow, upon sexual arousal (mimiced by pelvic nerve stimulation) without adversely affecting cardiovascular parameters, such as causing a significant hypotensive or hypertensive.

Preferably the compounds of the invention are administered for the treatment of FSD in the sexually stimulated patient (by sexual stimulation we mean to include visual, auditory or tactile stimulation). The stimulation can be before, after or during said administration.

Thus the compounds of the invention enhance the pathways/mechanisms that underlie sexual arousal in the female gentialia restoring or improving the sexual arousal response to sexual stimulation.

Thus a preferred embodiment provides the use of a compound of the invention in the preparation of a medicament for the treatment or prophyaxis of FSD in the stimulated patient.

For veterinary use, a compound of the invention, is administered as a suitably acceptable formulation in accordance with normal veterinary practice and the veterinary surgeon will determine the dosing regimen and route of administration which will be most appropriate for a particular animal.

The following formulation examples are illustrative only and are not intended to limit the scope of the invention . "Active ingredient" means a compound of the invention.

Formulation 1: A tablet is prepared using the following ingredients:

| | weight/mg |
|---|---|
| Active ingredient | 250 |
| Cellulose, microcrystalline | 400 |
| Silicon dioxide, fumed | 10 |
| Stearic acid | 5 |
| Total | 665 |

the components are blended and compressed to form tablets.

Formulation 2: An intravenous formulation may be prepared as follows:

| | |
|---|---|
| Active ingredient | 100mg |

(continued)

| | |
|---|---|
| Isotonic saline | 1,000ml |

Typical formulations useful for administering the compounds of the invention topically to the genitalia are as follows:

Formualtion 3: A spray

Active ingredient (1.0%) in isopropanol (30%) and water.

Formulation 4: A foam

Active ingredient, acetic acid glacial, benzoic acid, cetyl alcohol, methyl parahydroxybenzoate, phosphoric acid, polyvinyl alcohol, propylene glycol, sodium carboxymethylcellulose, stearic acid, diethyl stearamide, van Dyke perfume No. 6301, purified water and isobutane.

Formulation 5: A gel

Active ingredient, docusate sodium BP, isopropyl alcohol BP, propylene glycol, sodium hydroxide, carbomer 934P, benzoic acid and purified water.

Formulation 6: A Cream

Active ingredient, benzoic acid, cetyl alcohol, lavender, compound 13091, methylparaben, propylparaben, propylene glycol, sodium carboxymethylcellulose, sodium lauryl sulfate, stearic acid, triethanolmine, acetic acid glacial, castor oil, potassium hydroxide, sorbic acid and purified water.

Formulation 7: A pessary

Active ingredient, cetomacrogol 1000 BP, citric acid, PEG 1500 and 1000 and purified water.
The invention additionally includes:

(i) A pharmaceutical composition including a compound of the invention, together with a pharmaceutically acceptable excipient, diluent or carrier.
(ii) A compound of the invention for use as a medicament.
(iii) The use of a compound of the invention as a medicament for treating or preventing a condition for which a beneficial therapeutic response can be obtained by the inhibition of neutral endopeptidase.
(iv) The use of a compound of the invention as a medicament for treating or preventing hypoactive sexual desire disorder, sexual arousal disorder, orgasmic disorder or sexual pain disorder, preferably sexual arousal disorder, orgasmic disorder or sexual pain disorder, more preferably sexual arousal disorder.
(v) A method of treating FSD in a mammal including treating said mammal with an effective amount of a compound of the invention.
(vi) An FSD treating pharmaceutical composition comprising a compound of the invention together with a pharmaceutically acceptable excipient, diluent or carrier.
(vi) A compound of the invention for treating FSD.

The invention is illustrated by the following non-limiting examples in which the following abbreviations and definitions are used:

| | |
|---|---|
| Arbocel® | filter agent |
| br | broad |
| BOC | *tert*-butoxycarbonyl |
| CDI | carbonyldiimidazole |
| $\delta$ | chemical shift |
| d | doublet |
| $\Delta$ | heat |
| DCC | dicyclohexylcarbodiimide |

| | |
|---|---|
| DCM | dichloromethane |
| DMF | *N,N*-dimethylformamide |
| DMSO | dimethylsulfoxide |
| $ES^+$ | electrospray ionisation positive scan |
| $ES^-$ | electrospray ionisation negative scan |
| h | hours |
| HOBt | 1-hydroxybenzotriazole |
| HPLC | high pressure liquid chromatography |
| m/z | mass spectrum peak |
| min | minutes |
| MS | mass spectrum |
| NMR | nuclear magnetic resonance |
| q | quartet |
| s | singlet |
| t | triplet |
| Tf | trifluoromethanesulfonyl |
| TFA | trifluoroacetic acid |
| THF | tetrahydrofuran |
| TLC | thin layer chromatography |
| $TS^+$ | thermospray ionisation positive scan |
| WSCDI | 1 -(3-dimethylaminopropyl)-3-ethylcarbodiimide hydrochloride |

Melting points were determined using a Perkin Elmer DSC7 at a heating rate of 20°C/minute).

EXAMPLE 1

2-({1-[(Hexylamino)carbonyl]cyclopentyl}methyl)-4-methoxybutanoic acid

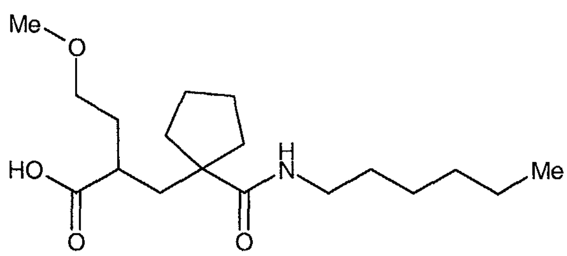

Trifluoroacetic acid (0.5ml) was added to the *tert*-butyl ester from preparation 9 (83mg, 0.2mmol) in dichloromethane (1 ml) at room temperature and stirred overnight. The reaction mixture was concentrated *in vacuo* and purified by chromatography (95:5 dichloromethane:methanol) to give the title product as a yellow oil, (71 mg, 0.2mmol), $^1H$ NMR ($CDCl_3$ 400MHz) δ: 0.8 (t, 3H), 1.2-1.3 (m, 6H), 1.45-1.55 (m, 4H), 1.6-1.7 (m, 4H), 1.7-1.75 (m, 1H), 1.8-1.95 (m, 2H), 1.95-2.1 (m, 2H), 2.15 (s, 2H), 2.4-2.5 (m, 1H), 3.15-3.2 (m, 2H), 3.25 (s, 3H), 3.35 (t, 2H), 6.1 (bs, 1H). LRMS: m/z 328 ($M+H^+$).

Compounds of formula I may be prepared by methods analogous to that of Example 1 from the precursor indicated (see Table 1.

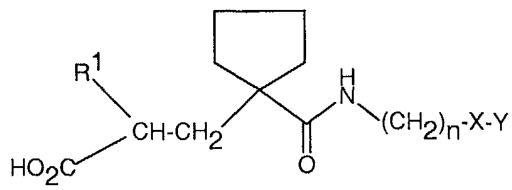

(I)

Table 1

| Ex | Prec | $R^1$ | $-(CH_2)_n$-X-Y | Data |
|---|---|---|---|---|
| 2 | Prep 10 | methoxy ethyl | 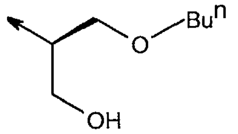 | $^1$H NMR ($CDCl_3$ 400MHz) δ: 0.9 (t, 3H), 1.3-1.35 (q, 2H), 1.45-1.7 (m, 11H), 1.85-2.0 (m, 2H), 2.0-2.4 (m, 3H), 2.5-2.6 (m, 1H), 3.3 (s, 3H), 3.3-3.5 (m, 3H), 3.5-3.55 (m, 1H), 3.55-3.6 (m, 1H), 3.7-3.75 (m, 1H), 4.0-4.1 (m, 1H), 6.4 (dd, 1H). LRMS: m/z 374 $(M-H^+)$. HRMS m/z 396.2357 ($C_{19}H_{35}NO_6Na$ requires 396.2356) |
| 3 | Prep 11 | methoxy ethyl | n-heptyl | $^1$H NMR ($CDCl_3$ 400MHz) δ: 0.8 (t, 3H), 1.1-1.35 (m, 8H), 1.45-1.7 (m, 10H), 1.75 (d, 2H), 1.85-1.95 (m, 2H), 1.95-2.1 (m, 2H), 2.4-2.5 (m, 1H), 3.15-3.25 (m, 2H), 3.3 (s, 3H), 3.4-3.5 (m, 2H), 6.2 (bs, 1H). LRMS: m/z 342 $(M+H^+)$. HRMS m/z 242.2656 ($C_{19}H_{35}NO_4$ requires 342.2639) |
| 4 | Prep 12 | methoxy ethyl | n-octyl | $^1$H NMR ($CDCl_3$ 400MHz) δ: 0.8 (t, 3H), 1.15-1.3 (m, 11H), 1.45-1.7 (m, 10H), 1.75 (dd, 2H), 1.85-1.95 (m, 2H), 1.95-2.1 (m, 2H), 2.4-2.5 (m, 1H), 3.15-3.2 (m, 2H), 3.3 (s, 3H), 3.35-3.4 (t, 2H), 6.1 (bs, 1H). LRMS: m/z 356 $(M+H^+)$. HRMS m/z 256.2803 ($C_{20}H_{37}NO_4$ requires 356.2796) |
| 5 | Prep 13 | methoxy ethyl | n-butoxy-n-propyl | $^1$H NMR ($CDCl_3$ 400MHz) δ: 0.9 (t, 3H), 1.3-2.0 (m, 18H), 2.4 (m, 1H), 3.2 (s, 3H), 3.25-3.45 (m, 8H), 4.8 (bs, 1H), 7.8 (bs, 1H). LRMS: m/z 356 $(M-H^-)$. HRMS m/z 358.2603 ($C_{19}H_{35}NO_5$ requires 358.2588). Found C, 62.81; H, 9.73; N, 3.80. $C_{19}H_{35}NO_5 \cdot 0.09$DCM requires C, 62.80; H, 9.71; N, 3.81% |
| 6 | Prep 14 | methoxy ethyl | 1-hydroxy-2-n-hexyl | $^1$H NMR ($CDCl_3$ 400MHz) δ: 0.8 (t, 3H), 1.25-1.35 (m, 4H), 1.4-1.75 (m, 10H), 1.85-2.1 (m, 4H), 2.1-2.2 (m, 1H), 2.4-2.55 (m, 1H), 3.25 (s, 3H), 3.3-3.5 (m, 2H), 3.7-4.0 (m, 1H), 4.2-4.4 (m, 1H), 5.8-5.95 (m, 1H). LRMS: m/z 344 $(M+H^+)$. HRMS m/z 344.2429 ($C_{18}H_{34}NO_5$ requires 344.2431). Found C, 57.88; H, 8.64; N, 3.36. $C_{18}H_{33}NO_5 \cdot 0.45$DCM requires C, 58.06; H, 8.95; N, 3.67% |
| 7 | Prep 15 | methoxy ethyl | 1-hydroxy-2-n-octyl | $^1$H NMR ($CDCl_3$ 400MHz) δ: 0.8 (t, 3H), 1.2-2.1 (m, 22H), 2.3 (m, 1H), 2.5 (m, 1H), 3.25 (s, 3H), 3.3 (m, 2H), 3.6 (m, 2H), 4.0 (m, 1H), 6.0 (m, 1H). LRMS: m/z 370 $(M-H^+)$. HRMS m/z 372.2737 ($C_{20}H_{37}NO_5$ requires 372.2745). |

| Ex | Prec | $R^1$ | $-(CH_2)_n$-X-Y | Data |
|---|---|---|---|---|
| 8 | Prep 16 | (S)-methoxy ethyl | n-butoxy-n-propyl | $^1$H NMR ($CDCl_3$ 400MHz) δ: 0.9 (t, 3H), 1.3-2.0 (m, 18H), 2.5 (m, 1H), 3.2 (s, 3H), 3.2-3.5 (m, 8H), 6.7 (s, 1H). LRMS: m/z 356 (M-H$^-$). HRMS m/z 358.2582 ($C_{19}H_{35}NO_5$ requires 358.2588). $[\alpha]_D$ +0.80 (EtOH, c 1.0). Found C, 62.93; H, 9.81; N, 3.66. $C_{19}H_{35}NO_5$·0.17DCM requires C, 61.91; H, 9.58; N, 3.77% |

The following Preparations describe the preparation of certain intermediates used in the preceding Examples.

PREPARATION 1

(2*S*)-3-[(2*E*)-2-Butenyloxy]-2-[(*tert*-butoxycarbonyl)amino]propanoic acid

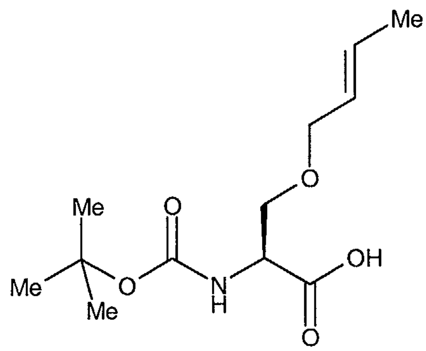

N-Boc-L-serine (30g, 146mmol) was added to a suspension of sodium hydride 80% in oil (8.77g, 242mmol) in *N,N*-dimethylformamide (600ml) at 0°C and stirred for 2 hours before the addition of crotyl bromide (19.71g, 0.146mmol). The reaction mixture was allowed to warm to room temperature overnight before been quenched by the addition of methanol (20ml). The reaction mixture was concentrated *in vacuo* and the residue dissolved in water (300ml) acidified with solid citric acid and extracted with ethyl acetate (4x100ml). The combined organic layers were washed with saturated brine (50ml), dried over magnesium sulphate to give the title product (33.4g, 129mmol); $^1$H NMR ($CDCl_3$ 400MHz) δ: 0.1.4 (s, 9H), 1.65 (d, 3H), 3.55-3.65 (m, 1H), 3.8-3.95 (m, 3H), 4.35-3.45 (m, 1H), 5.35 (d, 1H), 5.45-5.55 (m, 1H), 5.6-5.7 (m, 1H); LRMS: m/z 282 (M-H$^+$).

PREPARATION 2

(2*S*)-3-Butoxy-2-[(*tert*-butoxycarbonyl)amino]propanoic acid

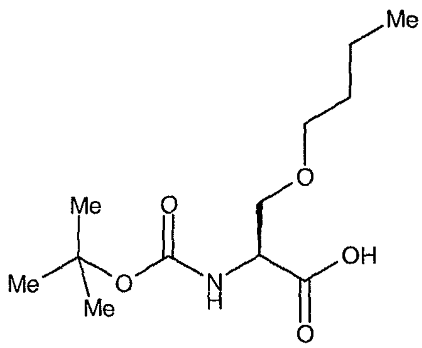

The product from preparation 1 (33g, 128mmol) and sodium hydrogencarbonate (12g) were dissolved in ethanol (500ml) and water (100ml) before being hydrogenated at 60 p.s.i. over 5% palladium on carbon (2g) at room temperature for 2 hours. The catalyst was removed by filtration and the filtrate concentrated *in vacuo.* The residue was dissolved in water (100ml) and acidified with solid citric acid before been extracted with ethyl acetate (3x50ml). The combined organic layers were washed with brine (50ml), dried over magnesium sulphate, filtered and concentrated *in vacuo.* The residue was purified by column chromatography 10:90:1 ethyl acetate:hexane:acetic acid (Rf=0.25) to give the title product (22.79g, 87.3mmol); $[\alpha]_D$-19° (MeOH, c 10.0); Found C, 54.32; H, 8.79; N, 5.53. $C_{12}H_{23}NO_5 \cdot 0.25H_2O$ requires C, 54.22; H, 9.91; N, 5.27%.

PREPARATION 3

Allyl (2*S*)-3-butoxy-2-[(*tert*-butoxycarbonyl)amino]propanoate

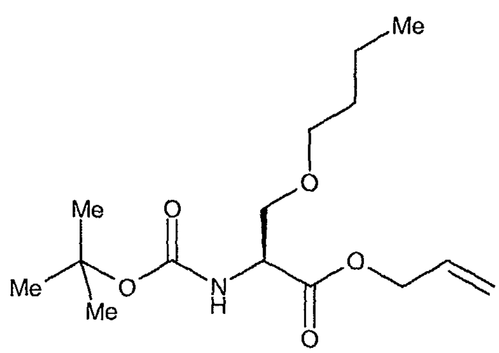

The product from preparation 2 (62.11g, 238mmol) and potassium carbonate (98.54g, 713mmol) were dissolved in *N,N*-dimethylformamide (150ml) and allyl bromide (30.85ml, 357mmol) in *N,N*-dimethylformamide (50ml) was added dropwise with cooling. The reaction mixture was allowed to warm to room temperature and stirred for 3 days before been concentrated *in vacuo.* The residue was dissolved in ethyl acetate (400ml) and water (600ml) and extracted with ethyl acetate (2x200ml). The combined organic extracts were washed with 1N hydrogen chloride solution (2x200ml), brine (200ml) and dried over magnesium sulphate, before been filtered and concentrated *in vacuo.* The crude reaction mixture was purified by column chromatography 1:10 diethyl ether:hexane then 1:3 diethyl ether:hexane (Rf = 0.9 1:1 diethyl ether:hexane) to give the title product (63.0g, 209mmol); $^1H$ NMR ($CDCl_3$ 400MHz) δ: 0.9 (t, 3H), 1.25-1.55 (m, 4H), 1.4 (s, 9H), 3.35-3.45 (m, 2H), 3.65 (dd, 1H), 3.85 (dd, 1H), 4.4-4.45 (m, 1H), 4.6-4.7 (m, 2H), 5.2-5.4 (m, 3H), 5.8-5.95 (m, 1H); $[\alpha]_D$-15° (MeOH, c 10.0); LRMS: m/z 302 ($M+H^+$); Found C, 59.94; H, 9.02; N, 4.64.

$C_{15}H_{27}NO_5$ requires C, 59.78; H, 9.03; N, 4.65%.

PREPARATION 4

Allyl (2*S*)-2-amino-3-butoxypropanoate hydrochloride

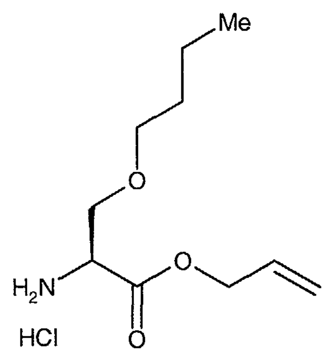

The product from preparation 3 (5.0g, 16.6mmol) was dissolved in ethyl acetate at 0°C and hydrogen chloride gas was bubbled through the solution for 1 hour. The reaction mixture stirred for a further 2 hours and concentrated *in vacuo* to give the title product (4.05g, 16.6mmol) as a crude oil; $^1H$ NMR ($CDCl_3$ 400MHz) δ: 0.8 (t, 3H), 1.3 (q, 2H), 1.5 (quin, 2H), 2.0 (m, 1H), 3.4-3.6 (m, 2H), 3.9-4.1 (m, 2H), 4.4 (bs, 1H), 4.7 (dq, 2H), 5.2 (d, 1H), 5.3 (d, 1H), 5.8-6.0 (m, 1H), 8.5 (bs, 3H); LRMS: m/z 148 ($M+H^+$).

PREPARATION 5

(2*R*)-2-Amino-3-butoxy-1-propanol

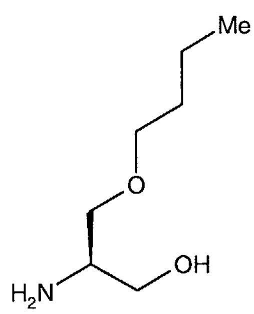

To a solution of the product from preparation 4 (4.05g, 16.6mmol) in tetrahydrofuran (100ml) at 0°C was added lithium aluminium hydride (1M solution in tetrahydrofuran (25ml), 25mmol). After 1 hour the reaction mixture was allowed to warm to room temperature and stirred for a further 2 hours. The reaction mixture was quenched by the addition of 1 M sodium hydroxide and filtered. The filtrate was concentrated *in vacuo* to give the title product (1.79g, 12.1mmol); $^1H$ NMR ($CDCl_3$ 400MHz) δ: 0.8 (t, 3H), 1.3 (q, 2H), 1.5-1.6 (m, 2H), 1.75 (bs, 2H), 3.0 (q, 1H), 3.35-3.45 (m, 4H), 3.45-3.6 (m, 2H).

PREPARATION 6

1-[-2-(*tert*-Butoxycarbonyl)-4-methoxybutyl]cyclopentanecarboxylic acid

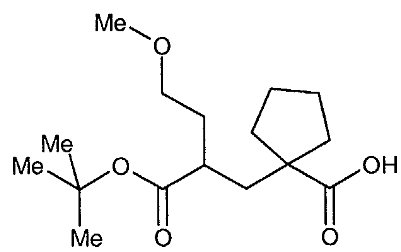

A solution of *tert*-butyl 3-(1-carboxycyclopentyl)propanoate (EP274234-B1 Example 35) (12g, 49.5mmol) in dry tetrahydrofuran (100ml) was added to a stirred solution of lithium diisopropylamide (130ml) in a mixture of hexane (52ml) and tetrahydrofuran (200ml) at -78°C under nitrogen. After 1 hour a solution of 2-bromoethyl methyl ether in tetrahydrofuran (100ml) was added maintaining the temperature at -78°C. The reaction mixture was allowed to warm up to room temperature overnight. The mixture was quenched with water (100ml) and acidified to pH 1 with 2M hydrochloric acid, and extracted with ethyl acetate (2x 150ml). The combined organic extracts were dried over magnesium sulphate and concentrated *in vacuo* to give the crude acid, which was chromatographed on silica. Elution with increasing proportions of methanol in dichloromethane (neat dichloromethane to 1:50) gave the title product (7.7g, 25.6mmol, 52%); Rf 0.3 methanol:dichloromethane 1:20; $^{1}H$ NMR ($CDCl_3$ 400MHz) δ: 1.4 (s, 9H), 1.4-1.7 (m, 7H), 1.75-1.95 (m, 2H), 2.0-2.15 (m, 3H), 2.3-2.4 (m, 1H), 3.3 (s, 3H), 3.3-3.4 (m, 2H); LRMS: m/z 299 ($M-H^+$).

PREPARATION 7

1-[(2*S*)-2-(*tert*-Butoxycarbonyl)-4-methoxybutyl]cyclopentanecarboxylic acid

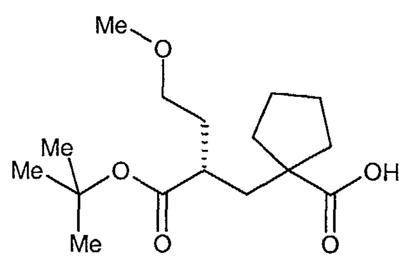

The racemic product from preparation 6 and (+)-pseudoephedrine were recrystallised nine times from hexane to give a white crystalline solid. This solid was dissolved in ethyl acetate washed with 0.5M hydrochloric acid, dried over magnesium sulphate and concentrated *in vacuo* to give the title product (typically 30% yield as a pale yellow oil in >90%ee by NMR analysis of the δ 3.3 peak of the (+)-pseudoephedrine salt); $^{1}H$ NMR ($CDCl_3$ 400MHz) δ: 1.4 (s, 9H), 1.4-1.7 (m, 7H), 1.75-1.9 (m, 2H), 2.0-2.15 (m, 3H), 2.35-2.45 (m, 1H), 3.3 (s, 3H), 3.3-3.4 (m, 2H); $[\alpha]_D$-5.2 (EtOH, c 1.2).

PREPARATION 8

1-[2-(*tert*-Butoxycarbonyl)-4-pentyl]-cyclopentane carboxylic acid

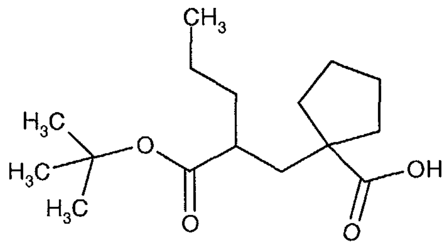

A mixture of 1-[2-(*tert*-butoxycarbonyl)-4-pentenyl]-cyclopentane carboxylic acid (EP 274234, Example 44) (23g, 81.5mmol) and 10% palladium on charcoal (2g) in dry ethanol (200ml) was hydrogenated at 30psi and room temperature for 18 hours. The reaction mixture was filtered through Arbocel®, and the filtrate evaporated under reduced pressure to give a yellow oil. The crude product was purified by column chromatography on silica gel, using ethyl acetate:pentane (40:60) as the eluant, to provide the title product as a clear oil, 21g, 91%; $^1H$ NMR ($CDCl_3$, 0.86 (t, 3H), 1.22-1.58 (m, 15H), 1.64 (m, 4H), 1.78 (dd, 1H), 2.00-2.18 (m, 3H), 2.24 (m, 1H); LRMS : m/z 283 (M-H).

PREPARATION 9

*tert*-butyl 2-({1-[(hexylamino)carbonyl]cyclopentyl}methyl)-4-methoxybutanoate

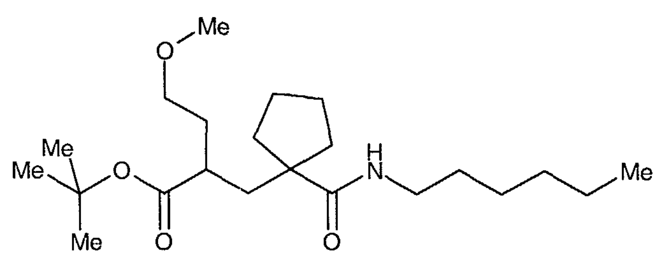

The racemic acid from preparation 6 (100mg, 0.33mmol), heptylamine (0.05ml, 0.33mmol), HOBt (45mg, 0.33mmol), WSCDI (64mg, 0.33mmol) and triethylamine (0.14ml, 1mmol) were stirred together in dichloromethane (2ml) at room temperature for 14 hours. The reaction mixture was diluted with dichloromethane (2ml) and washed with water (2ml). The organic layer was separated by filtering though a phase separation tube and concentrated *in vacuo.* The product was purified by chromatography using dichloromethane, then 99:1 dichloromethane:methanol, then 98:2 dichloromethane:methanol and then 95:5 dichloromethane:methanol to give the title product (83mg, 0.2mmol); $^1H$ NMR ($CDCl_3$ 400MHz) δ: 0.8 (t, 3H), 1.2-1.3 (m, 6H), 1.4 (s, 9H), 1.4-1.5 (m, 4H), 1.5-1.6 (m, 4H), 1.6-1.75 (m, 2H), 1.85-2.0 (m, 4H), 2.25-2.3 (m, 1H), 3.15 (q, 2H), 3.2 (m, 3H), 3.25 (t, 2H), 5.7 (bs, 1H); LRMS: m/z 384 ($M+H^+$).

Compounds of formula IVa, i.e. compounds of formula IV where Prot is *tert*-butyl, were prepared by methods similar to Preparation 9, from the precursors indicated (see Table 2).

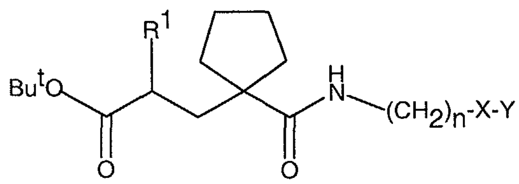

(IVa)

Table 2

| Prep | $R^1$ | $-(CH_2)_n$-X-Y | Prec. acid/amine | Analytical Data |
|---|---|---|---|---|
| 10 | methoxy ethyl | 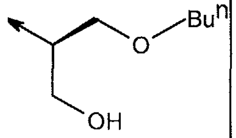 | Prep6/Prep5 | $^1H$ NMR ($CDCl_3$ 400MHz) $\delta$: 0.9 (t, 3H), 1.3-1.4 (q, 2H), 1.4 (s, 9H), 1.4-1.5 (m, 4H), 1.5-1.8 (m, 7H), 1.85-2.05 (m, 4H), 2.3-2.4 (m, 1H), 3.2 (d, 3H), 3.25-3.35 (m, 2H), 3.4-3.45 (m, 2H), 3.5-3.6 (m, 2H), 3.65-3.75 (m, 2H), 3.95-4.05 (m, 1H), 6.2 (dd, 1H). LRMS: m/z 430 ($M+H^+$). |
| 11 | methoxy ethyl | n-heptyl | Prep6/n-heptylamine (Aldrich) | $^1H$ NMR ($CDCl_3$ 400MHz) $\delta$: 0.8 (t, 3H), 1.2-1.3 (m, 8H), 1.4 (s, 9H), 1.4-1.5 (m, 4H), 1.5-2.05 (m, 10H), 2.3-2.4 (m, 1H), 3.15 (q, 2H), 3.2 (s, 3H), 3.3 (t, 2H), 5.7 (bs, 1H). LRMS: m/z 398 ($M+H^+$). |
| 12 | methoxy ethyl | n-octyl | Prep 6/n-octylamine (Aldrich) | $^1H$ NMR ($CDCl_3$ 400MHz) $\delta$: 0.8 (t, 3H), 1.1-1.3 (m, 10H), 1.4 (s, 9H), 1.4-1.5 (m, 4H), 1.5-1.6 (m, 5H), 1.55-1.75 (m, 2H), 1.8-2.0 (m, 3H), 2.2-2.3 (m, 1H), 3.15 (q, 2H), 3.2 (s, 3H), 3.3 (t, 2H), 5.7 (bs, 1H). LRMS: m/z 412 ($M+H^+$). |
| 13 | methoxy ethyl | n-butoxy-n-propyl | Prep 6/n-butoxy-n-propylamine (Aldrich) | $^1H$ NMR ($CDCl_3$ 400MHz) $\delta$: 0.9 (t, 3H), 1.3 (m, 2H), 1.4 (s, 9H), 1.5-1.65 (m, 9H), 1.7 (m, 4H), 2.0 (m, 3H), 2.3 (m, 1H), 3.2 (s, 3H), 3.3-3.5 (m, 8H), 7.5 (bs, 1H). LRMS: m/z 414 ($M-H^-$). |
| 14 | methoxy ethyl | 1-hydroxy-2-n-hexyl | Prep 6/1-hydroxy-2-n-hexylamine (Aldrich) | $^1H$ NMR ($CDCl_3$ 400MHz) $\delta$: 0.85 (t, 3H), 1.25-1.35 (m, 4H), 1.4-1.5 (m, 13H), 1.5-1.7 (m, 7H), 1.75-1.9 (m, 2H), 1.95-2.1 (m, 2H), 2.3-2.4 (m, 1H), 3.2-3.4 (m, 5H), 3.45-3.5 (m, 1H), 3.65-3.75 (m, 1H), 3.8-3.9 (m, 1H), 5.75 (bd, 1H). |

| Prep | $R^1$ | $-(CH_2)_n-X-Y$ | Prec. acid/amine | Analytical Data |
|---|---|---|---|---|
| 15 | methoxy ethyl | 1-hydroxy-2-n-octyl | Prep 6/ Prep 20 | $^1H$ NMR ($CDCl_3$ 400MHz) δ: 0.8 (m, 3H), 1.2 (m, 8H), 1.4 (s, 9H), 1.4-2.1 (m, 14H), 2.4 (m, 1H), 3.2 (s, 3H), 3.4 (m, 3H), 3.8 (m, 2H), 5.7 (s, 1H). LRMS: m/z 428 ($M+H^+$). |
| 16 | (S)-methoxy ethyl | n-butoxy-n-propyl | Prep 8/ n-butoxy-n-propylamine (Aldrich) | $^1H$ NMR ($CDCl_3$ 400MHz) δ: 0.9 (t, 3H), 1.3 (m, 2H), 1.4 (s, 9H), 1.5-2.0 (m, 16H), 2.3 (m, 1H), 3.2 (s, 3H), 3.3-3.5 (m, 8H), 6.4 (s, 1H). LRMS: m/z 414 ($M-H^-$). |

PREPARATION 17

2-[(*tert*-Butoxycarbonyl)amino}octanoic acid

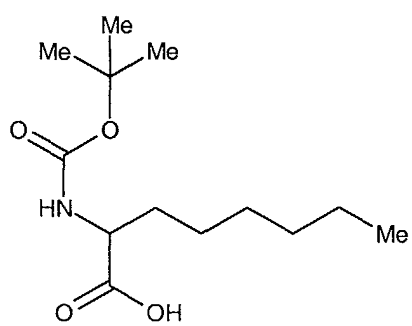

2-Aminocaprylic acid (1g, 6.3mmol) was taken up in 1 N NaOH (6.3mls, 6.3mmol) and dioxan (7mls) at 0°C under nitrogen and di-*tert*-butyl dicarbonate (1.51 g, 6.9mmol) added in one portion. The mixture was then stirred at 0°C for 15min, and then allowed to warm to room temperature overnight. The reaction was then concentrated *in vacuo* and water (15mls) added. The solution was acidified to pH1 using 2N HCl and the organics were then extracted with EtOAc. The dried ($MgSO_4$) organics were then concentrated *in vacuo* to give the title product (1.36g, 83%); $^1H$NMR (400MHz, $CDCl_3$) 0.90 (t, 3H), 1.30-1.40 (m, 8H), 1.40 (s, 9H), 1.70 (m, 2H), 4.00 (s, 1H); LRMS ES- 258 (M-H).

PREPARATION 18

Methyl-2-[(*tert*-butoxycarbonyl)amino}octanoate

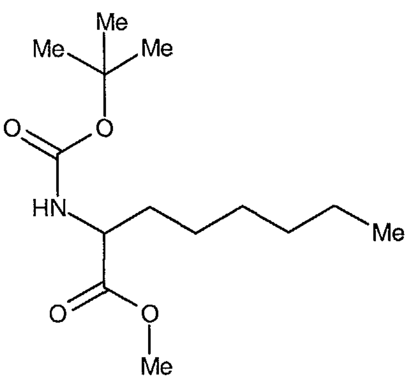

The acid from Preparation 17 (1.36g, 5.2mmol) was taken up in a mixture of methanol (5ml) and toluene (20ml) and stirred at room temperature under nitrogen. TMS diazomethane (2.6ml, 5.2mmol) was added dropwise over 5 mins, and the resulting pale yellow solution then stirred for 16h. The reaction was quenched carefully with acetic acid, concentrated *in vacuo* to afford an off-white solid, which was purified by column chromatography using 5% methanol in dichloromethane as eluant to give the title product (1.2g, 84%); $^1$HNMR (400MHz, $CDCl_3$) 0.80 (t, 3H), 1.20-1.40 (m, 8H), 1.40 (s, 9H), 1.50-1.70 (m, 2H), 3.70 (s, 3H), 4.20 (s, 1H), 4.90 (s, 1H); LRMS ES+ 274 (M+H).

PREPARATION 19

*Tert*-Butyl-(1-hydroxymethyl)-heptylcarbamate

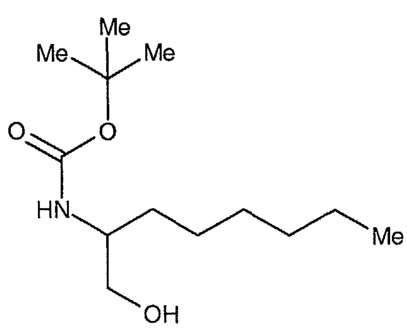

The ester from Preparation 18 (1.2g, 4.4mmol) was taken up in DCM (20ml) at -78°C under a nitrogen atmosphere and stirred as a 1M solution of DIBAL in DCM (26mls, 26mmol) was added dropwise over 10mins. The solution was then allowed to warm to room temperature overnight, before being quenched with a saturated solution of $NH_4Cl$. The resulting suspension was filtered through a plug of Arbocel and the filtrate then washed with water, dried ($MgSO_4$) and evaporated to a clear oil, which was purified by column chromatography using 5% MeOH in DCM as eluant to provide the title product (155mg, 14%); $^1$HNMR (400MHz, $CDCl_3$) 0.80 (m, 3H), 1.40 (s, 9H), 1.30-1.60 (m, 10H), 2.40 (s, 1H), 3.50 (m, 1H), 3.60 (m, 2H), 4.60 (s, 1H); LRMS ES+ 246 (M+H).

PREPARATION 20

2-Amino-1-octanol hydrochloride salt

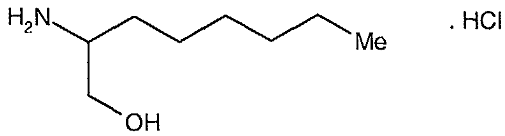

The carbamate from Preparation 19 (155mg, 0.6mmol) was taken up in DCM at 0°C and hydrogen chloride gas bubbled through for 15mins. After the passage of gas was ceased, the solution was stirred at 0°C for 15mins, and then at room temperature for 4h. The reaction was concentrated *in vacuo* to afford a white crystalline solid which was purified by trituration with ether to provide the title product (95mg, 91%); $^1$HNMR (400MHz, $CDCl_3$) 0.90 (m, 3H), 1.30-1.40 (m, 8H), 1.60 (m, 2H), 3.10 (s, 1H), 3.50-3.70 (m, 2H).

NEP Assay

The Preparation and Assay of Soluble Neutral Endopeptidase (NEP) from Canine, Rat, Rabbit and Human Kidney Cortex.

Soluble NEP is obtained from the kidney cortex and activity is assayed by measuring the rate of cleavage of the NEP substrate Abz-D-Arg-Arg-Leu-EDDnp to generate its fluorescent product, Abz-D-Arg-Arg.

Experimental Procedure:

1 Materials

All water is double de ionised.

1.1 Tissues:

| | |
|---|---|
| Human Kidney | IIAM (Pennsylvania. U.S.A.) |
| Rat Kidney | In house tissue supply |
| Rabbit Kidney | In house tissue supply |
| Canine Kidney | In house tissue supply |

1.2 Homogenisation medium:

100mM Mannitol and 20mM Tris @ pH 7.1

2.42g Tris (Fisher T/P630/60) is diluted in 1 litre of water and the pH adjusted to 7.1 using 6M HCl at room temperature. To this 18.22g Mannitol (Sigma M-9546) is added.

1.3 Tris buffer (NEP buffer):

50ml of 50mM Tris pH 7.4 (Sigma T2663) is diluted in 950ml of water.

1.4 Substrate (Abz-D-Arg-Arg-Leu-EDDnp):

Made to order from SNPE, and is stored as a powder at -20°C. A 2mM stock is made by gently re-suspending the substrate in Tris buffer, this should not be vortexed or sonicated. 600µl aliquots of the 2mM stock are stored at -20 for up to one month. (Medeiros, M.A.S., Franca, M.S.F. et al., (1997), Brazilian Journal of Medical and Biological Research, 30, 1157-1162).

1.5 Total product:

Samples corresponding to 100% substrate to product conversion are included on the plate to enable the % substrate turnover to be determined. The total product is generated by incubating 1ml of 2mM substrate with 20µl of enzyme stock for 24 hours at 37°C.

1.6 Stock solution:
A 300μM stock of Phosphoramidon (Sigma R7385) is made up in NEP buffer and stored in 50μl aliquots at -20.
1.7 Dimethyl sulphoxide (DMSO).
1.8 Magnesium Chloride -$MgCl_2.6H_2O$ (Fisher M0600/53).
1.9 Black 96 well flat bottom assay plates (Costar 3915).
1.10 Topseal A (Packard 6005185).
1.11 Centrifuge tubes

2 Specific Equipment

2.1 Sorvall RC-5B centrifuge (SS34 GSA rotor, pre-cooled to 4°C).
2.2 Braun miniprimer mixer.
2.3 Beckman CS-6R centrifuge.
2.4 Fluostar galaxy.
2.5 Wesbart 1589 shaking incubator.

3 Methods

3.1 Tissue Preparation
3.2 Dog, rat, rabbit, and human NEP is obtained from the kidney cortex using a method adapted from Booth, A. G. & Kenny, A.J. (1974) *Biochem. J.* 142, 575-581.
3.3 Frozen kidneys are allowed to thaw at room temperature and the cortex is dissected away from the medulla.
3.4 The cortex is finely chopped and homogenised in approximately 10 volumes of homogenisation buffer (1.2) using a Braun miniprimer (2.2).
3.5 Magnesium chloride (1.8) (20.3mg/gm tissue) is added to the homogenate and stirred in an ice-water bath for 15 minutes.
3.6 The homogenate is centrifuged at 1,500g (3,820rpm) for 12 minutes in a Beckman centrifuge (2.3) before removing the supernatant to a fresh centrifuge tube and discarding the pellet.
3.7 The supernatant is centrifuged at 15,000g (12,100rpm) for 12 minutes in a Sovall centrifuge (2.1) and the supernatant is discarded.
3.8 The pale pink layer on the top of the remaining pellet is removed and resuspended in homogenisation buffer containing magnesium chloride (9mg MgCl in 5ml buffer per 1g tissue).
3.9 The suspension is centrifuged at 2,200g (4,630rpm) for 12 minutes in a Beckman centrifuge (2.3) before discarding the pellet.
3.10 The supernatant is centrifuged at 15,000g (12,100rpm) for 12 minutes using the Sorvall centrifuge (2.1) and the supernatant is discarded.
3.11 The final pellet is resuspended in homogenisation buffer containing magnesium chloride (0.9mg MgCl in 0.5ml buffer per 1g tissue). A homogenous suspension is obtained using a Braun miniprimer (2.2). This is then frozen down in 100μl aliquots to be assayed for NEP activity.

4 Determination of NEP Activity

The activity of the previously aliquoted NEP is measured by its ability to cleave the NEP specific peptide substrate.

4.1 A 4% DMSO/NEP buffer solution is made (4mls DMSO in 96mls NEP buffer).
4.2 Substrate, total product, enzyme, and Phosphoramidon stocks are left on ice to thaw.
4.3 50μl of 4% DMSO/NEP buffer solution is added to each well.
4.4 The 2mM substrate stock is diluted 1:40 to make a 50μM solution. 100μl of 50μM substrate is added to each well (final concentration 25μM).
4.5 50μl of a range of enzyme dilutions is added to initiate the reaction (usually 1:100, 1:200, 1:400, 1:800, 1:1600, and 1:3200 are used). 50μl of NEP buffer is added to blank wells.
4.6 The 2mM total product is diluted 1:80 to make a 25μM solution. 200μl of 25μM product is added to the first four wells of a new plate.

4.7 Plates are incubated at 37oC in a shaking incubator for 60 minutes.
4.8 The 300µM Phosphoramidon stock is diluted 1:100 to 300nM. The reaction is stopped by the addition of 100µl 300nM Phosphoramidon and incubated at 37°C in a shaking incubator for 20 minutes before being read on the Fluostar (ex320/em420).

5 NEP Inhibition Assays

5.1 Substrate, total product, enzyme and Phoshoramidon stocks are left on ice to thaw.
5.2 Compound stocks are made up in 100% DMSO and diluted 1:25 in NEP buffer to give a 4% DMSO solution. All further dilutions are carried out in a 4% DMSO solution (4mls DMSO in 96mls NEP buffer).
5.3 50µl of compound in duplicate is added to the 96 well plate and 50µl of 4% DMSO/NEP buffer is added to control and blank wells (see appendix for plate layout). Alternatively see appendix for robotic dilutions.
5.4 The 2mM substrate stock is diluted 1:40 in NEP buffer to make a 50µM solution (275µl 2mM substrate to 10.73ml buffer is enough for 1 plate).
5.5 The enzyme stock diluted in NEP buffer (determined from activity checks).
5.6 The 2mM total product stock is diluted 1:80 in NEP buffer to make a 25µM solution. 200µl is added to the first four wells of a separate plate.
5.7 The 300µM Phosphoramidon stock is diluted 1:1000 to make a 300nM stock (11µl Phosphoramidon to 10.99ml NEP buffer.
5.8 To each well in the 96 well plate the following is added: Table: Reagents to be added to 96 well plate.

| | Compound/ DMSO | Tris Buffer | Substrate | NEP enzyme | Total product |
|---|---|---|---|---|---|
| Samples | 2µl compound | 50µl | 100µl | 50µl | None |
| Controls | 2µl DMSO | 50µl | 100µl | 50µl | None |
| Blanks | 2µl DMSO | 100µl | 100µl | None | None |
| Totals | 2µl DMSO | None | None | None | 200µl |

5.9 The reaction is initiated by the addition of the NEP enzyme before incubating at 37°C for 1 hour in a shaking incubator.
5.10 The reaction is stopped with 100µl 300nM Phosphoramidon and incubated at 37°C for 20 minutes in a shaking incubator before being read on the Fluostar (ex320/em420).

6 Calculations

The activity of the NEP enzyme is determined in the presence and absence of compound and expressed as a percentage.

% Control activity (turnover of enzyme) =

$$\frac{\text{Mean FU of controls - Mean FU of blanks}}{\text{Mean FU of totals - Mean FU of blanks}} \text{X } 100$$

% Activity with inhibitor =

$$\frac{\text{Mean FU of compound - Mean FU of blanks}}{\text{Mean FU of totals - Mean FU of blanks}} \text{X } 100$$

Activity expressed as % of control =

$$\frac{\text{\% Activity with inhibitor}}{\text{\% Control activity}} \text{X } 100$$

A sigmoidal dose-response curve is fitted to the % activities (% of control) vs compound concentration and IC50 values calculated using LabStats fit-curve in Excel.

ACE Assay

The Preparation and Assay of Soluble Angiotensin Converting Enzyme (Ace), from Porcine and Human Kidney Cortex.

Soluble ACE activity is obtained from the kidney cortex and assayed by measuring the rate of cleavage of the ACE substrate Abz-Gly-p-nitro-Phe-Pro-OH to generate its fluorescent product, Abz-Gly.

1 Materials

All water is double de ionised.

1.1 Human Kidney: IIAM (Pennsylvania. U.S.A.) or UK Human Tissue Bank (UK HTB)
1.2 Porcine kidney ACE Sigma (A2580)
1.3 Homogenisation buffer-1 100mM Mannitol and 20mM Tris @ pH 7.1
2.42g Tris (Fisher T/P630/60) is diluted in 1 litre of water and the pH adjusted to 7.1 using 6M HCl at room temperature. To this 18.22g Mannitol (Sigma M-9546) is added.
1.4 Homogenisation buffer-2
100mM Mannitol, 20mM Tris @ pH7.1 and 10mM $MgCl_2.6H_2O$ (Fisher M0600/53)
To 500ml of the homogenisation buffer 1 (1.4) 1.017g of $MgCl_2$ is added.
1.5 Tris buffer (ACE buffer).
50mM Tris and 300mM NaCl @ pH 7.4
50ml of 50mM Tris pH 7.4 (Sigma T2663) and 17.52g NaCl (Fisher S/3160/60) are made up to 1000ml in water.
1.6 Substrate (Abz-D-Gly-p-nitro-Phe-Pro-OH) (Bachem M-1100)
ACE substrate is stored as a powder at -20°C. A 2mM stock is made by gently re-suspending the substrate in ACE buffer, this must not be vortexed or sonicated. 400µl aliquots of the 2mM stock are stored at -20°C for up to one month.
1.7 Total product
Samples corresponding to 100% substrate to product conversion are included on the plate to enable the % substrate turnover to be determined (see calculations). The total product is generated by incubating 1ml of 2mM substrate with 20µl of enzyme stock for 24 hours at 37°C.
1.8 Stop solution.
0.5M EDTA (Promega CAS[6081/92/6]) is diluted 1:250 in ACE buffer to make a 2mM solution.
1.9 Dimethyl sulphoxide (DMSO).
1.10 Magnesium Chloride -$MgCl_2.6H_2O$ (Fisher M0600/53).
1.11 Black 96 well flat bottom assay plates (Costar 3915 or Packard).
1.12 Topseal A (Packard 6005185).
1.13 Centrifuge tubes

2 Specific Equipment

2.1 Sorvall RC-5B centrifuge (SS34 GSA rotor, pre-cooled to 4°C).
2.2 Braun miniprimer mixer.
2.3 Beckman CS-6R centrifuge.
2.4 BMG Fluostar Galaxy.
2.5 Wesbart 1589 shaking incubator.

3 Methods

3.1 Tissue Preparation
3.2 Human ACE is obtained from the kidney cortex using a method adapted from Booth, A.G. & Kenny, A.J. (1974) *Biochem. J.* 142, 575-581.
3.3 Frozen kidneys are allowed to thaw at room temperature and the cortex is dissected away from the medulla.
3.4 The cortex is finely chopped and homogenised in approximately 10 volumes of homogenisation buffer-1 (1.4) using a Braun miniprimer (2.2).

3.5 Magnesium chloride (1.11) (20.3mg/gm tissue) is added to the homogenate and stirred in an ice-water bath for 15 minutes.

3.6 The homogenate is centrifuged at 1,500g (3,820rpm) for 12 minutes in a Beckman centrifuge (2.3) before removing the supernatant to a fresh centrifuge tube and discarding the pellet.

3.7 The supernatant is centrifuged at 15,000g (12,100rpm) for 12 minutes in a Sovall centrifuge (2.1) and the supernatant is discarded.

3.8 The pale pink layer on the top of the remaining pellet is removed and resuspended in homogenisation buffer-2 (1.5) (5ml buffer per 1g tissue).

3.9 The suspension is centrifuged at 2,200g (4,630rpm) for 12 minutes in a Beckman centrifuge before discarding the pellet.

3.10 The supernatant is centrifuged at 15,000g (12,100rpm) for 12 minutes using the Sorvall centrifuge and the supernatant is discarded.

3.11 The final pellet is resuspended in homogenisation buffer-2 (0.5ml buffer per 1g tissue). A homogenous suspension is obtained using a Braun miniprimer. This is then frozen down in 100μl aliquots to be assayed for NEP activity.

4 Determination Of ACE Activity

The activity of the previously aliquoted ACE is measured by its ability to cleave the ACE specific peptide substrate.

Porcine ACE (1.2) is defrosted and resuspended in ACE buffer (1.6) at 0.004U/μl, this is frozen down in 50μl aliquots.

4.1 A 4% DMSO/ACE buffer solution is made (4mls DMSO in 96mls ACE buffer).

4.2 Substrate (1.7), total product (1.8) and enzyme (1.1, 1.2, 1.3), are left on ice to thaw.

4.3 50μl of 4% DMSO/ACE buffer solution is added to each well.

4.4 The 2mM substrate stock is diluted 1:100 to make a 20μM solution. 100μl of 20μM substrate is added to each well (final concentration in the assay 10μM).

4.5 50μl of a range of enzyme dilutions is added to initiate the reaction (usually 1:100, 1:200, 1:400, 1:800, 1:1600, and 1:3200 are used). 50μl of ACE buffer is added to blank wells.

4.6 The 2mM total product is diluted 1:200 to make 10μM solution. 200μl 10μM product is added to the first four wells of a new plate.

4.7 Plates are incubated at 37°C in a shaking incubator for 60 minutes.

4.8 The enzyme reaction is stopped by the addition of 100μl 2mM EDTA in ACE buffer and incubated at 37°C in a shaking incubator for 20 minutes before being read on the BMG Fluostar Galaxy (ex320/em420).

5 ACE Inhibition Assays

5.1 Substrate, total product, and enzyme stocks are left on ice to thaw.

5.2 Compound stocks are made up in 100% DMSO and diluted 1:25 in ACE buffer to give a 4% DMSO solution. All further dilutions are carried out in a 4% DMSO/ACE buffer solution (4mls DMSO in 96mls ACE buffer).

5.3 50μl of compound, in duplicate, is added to the 96 well plate and 50μl of 4% DMSO/ACE buffer is added to control and blank wells (see appendix-1 for plate layout).

5.4 Steps 5.2 and 5.3 can be carried out either by hand or using the Packard multiprobe robots (see appendix-2 for details)

5.5 The 2mM substrate stock is diluted 1:100 in ACE buffer to make a 20μM solution (10μM final concentration in the assay) (110μl of 2mM substrate added to 10.89ml buffer is enough for 1 plate).

5.6 The enzyme stock is diluted in ACE buffer, as determined from activity checks (4.0).

5.7 The 2mM total product stock is diluted 1:200 in ACE buffer to make a 10μM solution. 200μl is added to the first four wells of a separate plate.

5.8 The 0.5mM EDTA stock is diluted 1:250 to make a 2mM stock (44μl EDTA to 10.96ml ACE buffer).

5.9 To each well of the 96 well plate the following reagents are added:

Table 1:

| Reagents added to 96 well plate. | | | | | |
|---|---|---|---|---|---|
| | Compound/ DMSO | Tris Buffer | Substrate | ACE enzyme | Total product |
| Samples | 2µl compound | 50µl | 100µl | 50µl | None |
| Controls | 2µl DMSO | 50µl | 100µl | 50µl | None |
| Blanks | 2µl DMSO | 100µl | 100µl | None | None |
| Totals | 2µl DMSO | None | None | None | 200µl |

5.10 50µl of the highest concentration of each compound used in the assay is added in duplicate to the same 96 well plate as the totals (5.7). 150µl of ACE buffer is added to determine any compound fluorescence.
5.11 The reaction is initiated by the addition of the ACE enzyme before incubating at 37°C for 1 hour in a shaking incubator.
5.12 The reaction is stopped by the addition of 100µl 2mM EDTA and incubated at 37°C for 20 minutes in a shaking incubator, before being read on the BMG Fluostar Galaxy (ex320/em420).

6 Calculations

The activity of the ACE enzyme is determined in the presence and absence of compound and expressed as a percentage. (FU = Fluorescence units)

(i)

$$\underline{\text{\% Control activity (turnover of enzyme)}} = \frac{\text{Mean FU of controls - Mean FU of blanks}}{\text{Mean FU of totals - Mean FU of blanks}} \times 100$$

(ii)

$$\underline{\text{\% Activity with inhibitor =}}\quad \frac{\text{Mean FU of compound - Mean FU of blanks}}{\text{Mean FU of totals - Mean FU of blanks}} \times 100$$

(iii)

$$\underline{\text{Activity expressed as \% of control =}}\quad \frac{\text{\% Activity with inhibitor}}{\text{\% Control activity}} \times 100$$

$$\text{or } \frac{\text{Mean FU of compound - Mean FU of blanks}}{\text{Mean FU of controls - Mean FU of blanks}} \times 100$$

(iv)

% Inhibition = 100 - % control

(v) For fluorescent compounds the mean FU of blanks containing compound (5.10) is deducted from the mean FU of compound values used to calculate the % Activity.

A sigmoidal dose-response curve is fitted to the % activities (% of control) vs compound concentration and $IC_{50}$ values calculated using LabStats fit-curve in Excel.
The specific examples herein had an IC50 against NEP of less than 5000nM.

In addition many of the examples tested had a selectivity for NEP over ACE of at least 300 fold.

## Claims

1. A compound of formula (I), pharmaceutically acceptable salts, solvates or prodrugs thereof;

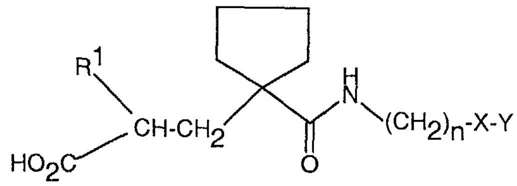

(I)

wherein

| | |
|---|---|
| $R^1$ | is $C_{1-6}$alkyl which may be substituted by one or more substituents, which may be the same or different, selected from the list: halo, hydroxy, $C_{1-6}$alkoxy, $C_{1-6}$hydroxyalkoxy, $C_{1-6}$alkoxy$C_{1-6}$alkoxy, carbocyclyl, carbocyclyloxy, $C_{1-4}$alkoxycarbocyclyloxy, heterocyclyl, heterocyclyloxy, $-NR^2R^3$, $-NR^4COR^5$, $-NR^4SO_2R^5$, $-CONR^2R^3$, $-S(O)_pR^6$, $-COR^7$ and $-CO_2(C_{1-4}$alkyl); or $R^1$ is carbocyclyl or heterocyclyl, each of which may be substituted by one or more substituents from said list, which substituents may be the same or different, which list further includes $C_{1-6}$alkyl; or $R^1$ is $C_{1-6}$alkoxy, $-NR^2 R^3$ or $-NR^4SO_2R^5$; wherein |
| $R^2$ and $R^3$, | which may be the same or different, carbocyclyl or heterocyclyl (each of which may be substituted by $C_{1-4}$alkyl, hydroxy or $C_{1-4}$alkoxy), or are hydrogen or $C_{1-4}$alkyl; or $R^2$ and $R^3$ together with the nitrogen to which they are attached form a pyrrolidinyl, piperidino, morpholino, piperazinyl or *N*-($C_{1-4}$ alkyl)piperazinyl group; |
| $R^4$ | is H or $C_{1-4}$alkyl; |
| $R^5$ | is $C_{1-4}$alkyl, $CF_3$, aryl, ($C_{1-4}$ alkyl)aryl, ($C_{1-4}$alkoxy)aryl, heterocyclyl, $C_{1-4}$alkoxy or $-NR^2R^3$; |
| $R^6$ | is $C_{1-4}$alkyl, aryl, heterocyclyl or $NR^2R^3$; and |
| $R^7$ | is $C_{1-4}$alkyl, $C_{3-7}$cycloalkyl, aryl or heterocyclyl; |
| p | is 0, 1, 2 or 3; |
| n | is 2 to 6; |
| X | is oxygen, sulfur or $-CH_2-$; |
| Y | is $C_{1-6}$alkyl which may be branched- or straight-chain, and may be independently substituted by one or more halo, $C_{1-4}$alkoxy, hydroxy or $C_{3-7}$cycloalkyl; wherein the linkage $-(CH_2)_n-$ and the linkage when X is $-CH_2-$, may be substituted by $C_{1-4}$alkyl, $C_{1-4}$haloalkyl, hydroxy, $C_{1-4}$alkoxy, $C_{1-4}$haloalkoxy, hydroxy$C_{1-4}$alkyl, $C_{1-4}$alkoxy$C_{1-4}$alkyl, $C_{1-4}$haloalkoxy$C_{1-4}$alkyl, $C_{3-7}$cycloalkyl or $C_{3-7}$cycloalkyl$C_{1-4}$alkyl. |

2. A compound according to claim 1, pharmaceutically acceptable salts, solvates or prodrugs thereof; wherein $R^1$ is $C_{1-6}$alkyl, $C_{1-6}$alkoxy, $C_{1-6}$alkoxy($C_{1-3}$)alkyl, $C_{1-6}$alkoxy$C_{1-6}$alkoxy$C_{1-3}$alkyl or $C_{1-6}$alkyl substituted with phenyl, optionally substituted by one or more alkyl, alkoxy, alkylthio, halogen, or phenyl (which phenyl may be independently substituted by one or more alkyl, alkoxy, alkylthio or halogen).

3. A compound according to claim 2, pharmaceutically acceptable salts, solvates or prodrugs thereof; wherein $R^1$ is $C_{1-6}$alkyl, $C_{1-6}$alkoxy, $C_{1-6}$alkoxy($C_{1-3}$)alkyl or $C_{1-6}$alkoxy$C_{1-6}$alkoxy$C_{1-3}$alkyl.

4. A compound according to claim 3, wherein $R^1$ is $C_{1-4}$alkyl or $C_{1-6}$alkoxy($C_{1-3}$)alkyl.

5. A compound according to any preceding claim, pharmaceutically acceptable salts, solvates or prodrugs thereof of formula Ia

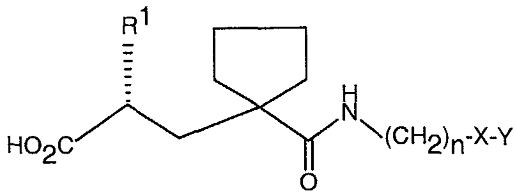

(Ia)

.

**6.** A compound selected from the group:

2-{[1-({[2-butoxy-1-(hydroxymethyl)ethyl]amino}carbonyl)cyclopentyl]-methyl}-4-methoxybutanoic acid;
4-methoxy-2-({1-[(octylamino)carbonyl]cyclopentyl}methyl)butanoic acid;
2-({1-[(heptylamino)carbonyl]cyclopentyl}methyl)-4-methoxybutanoic acid;
2-[(-{[(3-butoxypropyl)amino]carbonyl}cyclopentyl)methyl]-4-methoxybutanoic acid;
2-{[1-({[1-hydroxymethyl)heptyl]amino}carbonyl)cyclopentyl]methyl}-4-methoxybutanoic acid; and pharmaceutically acceptable salts, solvates or prodrugs thereof.

**7.** A pharmaceutical composition comprising a compound of formula (I) as claimed in claims 1-6 or pharmaceutically acceptable salts, solvates or prodrugs thereof; and a pharmaceutically acceptable diluent or carrier.

**8.** A compound of formula (I) as claimed in any one of claims 1-6 or pharmaceutically acceptable salts, solvates or prodrugs thereof for use as a medicament.

**9.** The use of a compound of formula (I) as claimed in any one of claims 1-6 or pharmaceutically acceptable salts, solvates or prodrugs thereof in the preparation of a medicament for the prevention or treatment of a condition for which a beneficial response is obtained by the inhibition of neutral endopeptidase.

**10.** The use as claimed in claim 9 where the condition is selected from: hypertension, heart failure, angina, renal insufficiency, acute renal failure, cyclical oedema, Menières disease, hyperaldosteroneism (primary and secondary), hypercalciuria, glaucoma, menstrual disorders, preterm labour, pre-eclampsia, endometriosis, reproductive disorders, asthma, inflammation, leukemia, pain, epilepsy, affective disorders, dementia and geriatric confusion, obesity, gastrointestinal disorders, wound healing, septic shock, the modulation of gastric acid secretion, the treatment of hyperreninaemia, cystic fibrosis, restenosis, diabetic complications, athereosclerosis, female sexual dysfunction (FSD) and male sexual dysfunction.

**11.** The use as claimed in claims 9 and 10 wherein the condition is selected from female sexual dysfunction and male sexual dysfunction.

**12.** The use as claimed in any one of claims 9-11 wherein the condition is female sexual arousal dysfunction.

**13.** A method of treating or preventing a condition in a mammal for which a beneficial response is obtained by the inhibition of neutral endopeptidase which comprises administering a therapeutically effective amount of a compound of formula (I) as claimed in claims 1-6 or pharmaceutically acceptable salts, solvates or prodrugs thereof to a patient in need of such treatment.

**14.** A method as claimed in claim 13 where the condition is selected from: hypertension, heart failure, angina, renal insufficiency, acute renal failure, cyclical oedema, Menières disease, hyperaldosteroneism (primary and secondary), hypercalciuria, glaucoma, menstrual disorders, preterm labour, pre-eclampsia, endometriosis, reproductive disorders, asthma, inflammation, leukemia, pain, epilepsy, affective disorders, dementia and geriatric confusion, obesity, gastrointestinal disorders, wound healing, septic shock, the modulation of gastric acid secretion, the treatment of hyperreninaemia, cystic fibrosis, restenosis, diabetic complications, athereosclerosis, female sexual dysfunction (FSD) and male sexual dysfunction.

**15.** A method as claimed in claims 13 and 14 where the condition is selected from female sexual dysfunction and male

sexual dysfunction.

**16.** A method as claimed in claims 13, 14 and 15 where the condition is female sexual arousal dysfunction.